(19)

**Europäisches Patentamt**

**European Patent Office**

**Office européen des brevets**

(11) **EP 4 112 728 A1**

(12) **EUROPEAN PATENT APPLICATION**

(43) Date of publication:
**04.01.2023 Bulletin 2023/01**

(51) International Patent Classification (IPC):
**C12N 15/10** (2006.01)     **C07K 1/04** (2006.01)
**G01N 33/68** (2006.01)

(21) Application number: **21182971.8**

(22) Date of filing: **30.06.2021**

(52) Cooperative Patent Classification (CPC):
**C12N 15/1034; C12N 15/70; C12Q 1/68;**
**C40B 40/02; C40B 40/08; G01N 33/6845**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **ETH Zürich**
**8092 Zürich (CH)**

(72) Inventors:
• **Schmitt, Steven**
  **4055 Basel (CH)**

• **Koch, Philipp**
  **4058 Basel (CH)**
• **Held, Martin**
  **8006 Zürich (CH)**
• **Panke, Sven**
  **8057 Zürich (CH)**

(74) Representative: **Eisenführ Speiser**
**Patentanwälte Rechtsanwälte PartGmbB**
**Arnulfstraße 27**
**80335 München (DE)**

Remarks:
The complete document including Reference
Table(s) and the Sequence Listing(s) can be
downloaded from the EPO website

(54) **METHOD FOR IDENTIFYING TARGET-BINDING PEPTIDES**

(57)     The present invention relates to a method for identifying peptides that specifically bind to a target molecule. The method may be used to identify both ribosomal and non-ribosomal peptides. With the method of the invention, peptides for the treatment of infectious and non-infectious diseases may be identified. Further, the invention relates to DNA libraries that can be used in the claimed method.

**EP 4 112 728 A1**

**Description**

The present invention relates to a method for identifying peptides that specifically bind to a target molecule. The method may be used to identify both ribosomal and non-ribosomal peptides. With the method of the invention, peptides for the treatment of infectious and non-infectious diseases may be identified. Further, the invention relates to DNA libraries that can be used in the claimed method.

**BACKGROUND**

Natural compounds are fundamental for drug discovery as they provide the biological relevance and structural diversity required to identify drug-like pharmacophores. Owing to their high structural complexity and their ability to penetrate tissues and membranes, peptides are becoming increasingly important for many therapeutic areas.

During the past decade, peptides have gained a wide range of applications in medicine and biotechnology, and therapeutic peptide research is currently experiencing a renaissance for commercial reasons. Currently, there are more than 60 US Food and Drug Administration (FDA)-approved peptide medicines on the market and this is expected to grow significantly, with approximately 140 peptide drugs currently in clinical trials and more than 500 therapeutic peptides in preclinical development.

Especially antimicrobial therapies have a very strong demand for novel compounds due to rising antimicrobial resistance. Although about 3,000 natural antimicrobial peptides have already been discovered, advances in genome sequencing and mining provide an ever-increasing number of peptides with elusive functions. Peptides also hold great promise for use in the treatment and/or prevention of non-infectious diseases. Due to their structural variability, peptides may for example be designed to interrupt underlying mechanisms of a disease, for example by blocking abnormal protein-protein interactions or by inhibiting overactive enzymes.

Given the large sequence space of natural peptides, the simplicity to modify existing peptides and the availability of computational methods to design artificial peptides, there is a need in the art for improved methods to identify peptides that specifically bind to target molecules. Accordingly, it is an objective of the present invention to provide improved methods for the identification of target-binding peptides.

**SUMMARY OF THE INVENTION**

The above technical problem is solved by the present invention as defined in the claims and as described herein below.

The inventors have developed a method that allows for the identification of target-binding peptides in a host cell. Using host cells for the synthesis of peptides from DNA libraries encoding the peptides as such and/or complexes of non-ribosomal peptide biosynthesis enzymes obviates costly chemical synthesis and purification of peptides, thereby significantly reducing the cost and increasing the throughput compared to methods known in the art.

The method of the invention is highly versatile, as it allows the identification of antimicrobial peptides, as well as peptides that may be used in the treatment of non-infectious diseases. Furthermore, the method of the invention allows identifying multiple target-binding peptides in a single experiment. In certain embodiments, the method of the invention further allows to simultaneously identify a peptide and its respective target molecule.

Accordingly, in a particular embodiment, the invention relates to a method for identifying a peptide that binds specifically to a target molecule, the method comprising the steps of:

(a) introducing a DNA library into a plurality of host cells, the DNA library comprising at least two members, wherein each of the at least two DNA library members comprises a nucleic acid sequence encoding

(i) a peptide candidate; or
(ii) a candidate complex of non-ribosomal peptide biosynthesis enzymes;

wherein the nucleic acid sequence encoding the peptide candidate or the candidate complex of non-ribosomal peptide biosynthesis enzymes is operably linked to an inducible promoter;
wherein the host cell comprises the target molecule, and wherein specific binding of a peptide to said target molecule induces a change in a measurable output signal;

(b) culturing the plurality of host cells obtained in step (a);
(c) inducing the synthesis of the peptide candidates and/or the candidate complex of non-ribosomal peptide biosynthesis enzymes in the plurality of host cells cultured in step (b);
(d) collecting a first sample at a first time point from the culture of step (b) before the induction step (c) and collecting a second sample at a second time point from the culture of step (b) after the induction step (c);
(e) determining the abundance of measurable output signals in the samples taken at the first time point and at the second time point for at least one peptide candidate or at least one candidate complex of non-ribosomal peptide biosynthesis enzymes; and
(f) identifying

(i) a peptide candidate to be a target binding peptide if the abundance of measurable output signals generated by said peptide candidate is different between the first time point and the second time point; and/or
(ii) a candidate complex of non-ribosomal peptide biosynthesis enzymes to synthesize a target binding peptide if the abundance of measurable output signals generated by said candidate complex of non-ribosomal peptide biosynthesis enzymes is different between the first time point and the second time point.

That is, the inventors have developed a method for identifying peptides that specifically bind to a target molecule. The method of the invention is characterized in that measurable output signals are either generated or inhibited upon specific binding of a peptide to a target molecule. For at least one peptide candidate encoded in a DNA library, the abundance of measurable output signals in a sample is determined at two or more time points. The first time point is always prior to the synthesis of the peptide candidate in a host cell. That is, at the first time point, the abundance of measurable output signals is determined in the absence of any peptide. For comparison, the abundance of measurable output signals is again determined at one or more time points after inducing the expression of a peptide candidate from a DNA library. By comparing the abundance of measurable output signals before and after the induction step, it can then be determined whether a peptide candidate specifically binds to a target molecule.

Within the present invention, the first sample is collected before the synthesis of the peptide candidates in the host cells. Preferably, the first sample is collected before the induction step. That is, in embodiments where a chemical inducer is used, the first sample is preferably collected before the chemical inducer is added to the cells. However, the skilled person is aware that the addition of an inducer does not instantly result in gene expression, let alone a physiological response of the host cells. Accordingly, it is to be understood that a sample that has been collected "before the induction step" also includes samples that have been collected directly after the addition of an inducer. That is, a sample that has been collected "before the induction step" also includes samples that have been collected up to 5 minutes, up to 10 minutes, up to 15 minutes or up to 20 minutes after the addition of the inducer to the cells.

Preferably, the method of the invention is based on a readout method that allows linking the abundance of measurable output signals to the sequence of a peptide candidate encoded in the DNA library. That is, the abundance of measurable output signals may be determined, without limitation, by next-generation sequencing, which allows quantifying a measurable output signal by counting the reads in a sample and, at the same time, allows assigning the measurable output signal to a specific peptide by determining the DNA sequence of the peptide or of the complex of non-ribosomal peptide biosynthesis enzymes encoded in said host cell. Most preferably, the method of the invention allows to individually determining an abundance of measurable output signals for each peptide candidate in a sample.

Within the present invention, either a higher or a lower abundance of measurable output signals in response to the expression of a candidate peptide may be indicative of a specific binding of said peptide candidate to a target molecule. For that, it has to be noted that the skilled person is capable of designing reporter systems that either result in an increase or in a decrease in the abundance of measurable output signals upon specific binding of a peptide candidate to a target molecule.

For example, a host cell may be engineered such that a measurable output signal is detectable when a target molecule is present in its native form, i.e. not bound to a candidate peptide. In this case, a reporter system may be designed such that specific binding of a peptide candidate to said target molecule inhibits the measurable output signal, which would consequently result in a lower abundance of a measurable output signals in a sample for said peptide candidate at the second time point (after expression of the candidate peptide) in comparison to the first time point (before expression of the candidate peptide).

Alternatively, a host cell may be engineered such that a measurable output signal is generated upon specific binding of a peptide candidate to a target molecule. In such cases, specific binding of a peptide candidate to a target molecule would result in a higher abundance of measurable output signals in a sample for said peptide candidate at the second time point (after expression of the candidate peptide) compared to the first time point (before expression of the candidate peptide).

In a particular embodiment, the invention relates to the method according to the invention, wherein two or more samples are collected after the induction step (c), and wherein the steps (e) and (f) are performed with each sample.

As mentioned above, the method according to the invention comprises at least two sampling steps, one before or directly after inducing the expression of a DNA library and one after inducing the expression of a DNA library. However, it is to be understood that more than one sample may be collected and analyzed after the induction step.

Collecting and analyzing two or more samples after the induction step may be advantageous, as it allows analyzing the response to a peptide in more detail. For example, taking multiple samples after the induction step may help to obtain a more robust readout and may allow tracking the response of a host cell to a specific peptide overtime. For example, collecting and analyzing two or more samples after the induction step may allow determining how rapidly the synthesis of a ribosomal or non-ribosomal peptide is translated into a measurable output signal. Further, collecting and analyzing two or more samples after the induction step may allow tracking the effect of a peptide over a longer time period. For example, in embodiments where the measurable output signal is cell viability, collecting and analyzing multiple samples after the induction step may allow recording a growth curve. In such embodiments, comparing growth curves of populations of host cells expressing different peptides may help identifying very efficient binders and/or may allow to draw conclusion on the mode-of-action of a peptide candidate. For example, it has been shown by the inventors that peptides that target the membrane of a host cell are act faster than peptides that act on intracellular targets.

The method of the present invention may comprise the collection and analysis of 1, 2, 3, 4, 5, 6, 7, 8, 9, 10 or more than 10 samples after the induction step. Preferably all samples are collected and analyzed by similar means.

In a particular embodiment, the invention relates to the method according to the invention, wherein the target molecule is (i) an essential component of the host cell; or (ii) a component of a cell, preferably a mammalian cell, in particular wherein said component is comprised in or functionally linked to a recombinant biosensor.

Within the present invention, a measurable output signal is generated or inhibited upon binding of a peptide candidate to a target molecule. Thus, the target molecule may be any molecule that either directly or indirectly induces or inhibits the generation of a measurable output signal in a host cell when bound by a peptide.

In certain embodiments, the target molecule is an essential component of a host cell. In particular, the target molecule may be an essential component of a host cell which cannot perform its essential function when specifically bound by a peptide. In such embodiments, specific binding of a peptide to a target molecule would result in growth arrest and, eventually, cell death. Thus, in embodiments where the target molecule is an essential component of a host cell, the measurable output signal preferably correlates with cell viability. In these embodiments, a peptide is determined to specifically bind to a target molecule, if the abundance of measurable output signal after the induction step is lower than before the induction step.

Accordingly, the method of the invention may be used to identify peptides that inhibit growth of a host cell. If the host cell is a microbial host cell, the method of the invention may be used to identify antimicrobial peptides. One advantage of the method of the invention is that multiple growth inhibiting and/or antimicrobial peptides may be identified in a single experiment. A further advantage of the method of the invention is that growth inhibiting and/or antimicrobial peptides may be identified directly in a pathogenic organism without prior knowledge of the host organisms or of a particular target molecule.

In certain embodiments, the target molecule may be a non-essential cell component. In order to receive a measurable output signal, the non-essential cell component is preferably comprised in a recombinant biosensor or functionally linked to a recombinant biosensor. In such embodiments, specific binding of a peptide to the target molecule will activate the recombinant biosensor such that it induces or inhibits the generation of a measurable output signal.

It is to be understood that the target molecule does not necessarily have to be comprised in the biosensor. In certain embodiments, the target molecule and the biosensor may be separate entities. However, the binding status of the target molecule is functionally linked to the activity of the biosensor. For example, the target molecule may be a cleavage enzyme, such as a protease, and the biosensor may have a cleavage site that is specifically recognized by the cleavage enzyme. In such embodiments, the cleavage enzyme will degrade the biosensor in the absence of a peptide, thereby diminishing the activity of the biosensor. Such systems may be used for the identification of peptides that inhibit the activity of the cleavage enzyme, such as novel protease inhibitors. In particular, inhibiting the activity of a cleavage enzyme will prevent the cleavage of the biosensor, which in turn will induce a change in the abundance of a measurable output signal.

The cell component may be any component of a cell that can be specifically bound by a peptide. In certain embodiments, the cell component is a component of a mammalian cell. In certain embodiments, the cell component of a mammalian cell may be involved in the pathogenesis of a disease. Thus, the method of the invention may be used for the identification of peptides that can be used in the prevention and/or treatment of a disease. In contrast to the embodiments where the target molecule is an endogenous essential component of a host cell, the screening for peptides that bind to non-endogenous cell components usually requires the use of an engineered cell and/or an engineered biosensor. Examples of engineered biosensors that allow identification of peptides that specifically bind to non-essential cell components are provided below.

In a particular embodiment, the invention relates to a method according to the invention, wherein the measurable output signal corresponds to (i) cell viability; or (ii) expression of a reporter gene.

In certain embodiments, the measurable output signal corresponds to cell viability of a host cell. Where the target molecule is an endogenous essential component of a host cell, specific binding of a peptide to the target molecule results in the dilution of host cells encoding said peptide in a sample. In such embodiments, the measurable output signal may be the abundance of next generation sequencing reads that encode the growth inhibiting peptide. Since the copy number of library elements in a host cell is expected to be constant, the number of reads corresponding to a particular library member directly correlates with the number of cells in a sample that express the peptide encoded by said library member.

When the target molecule is comprised in or functionally linked to a recombinant biosensor, the measurable output signal preferably correlates to the expression of a reporter gene. The skilled person is aware that sensor systems may be designed in a way such that activity of a biosensor either induces or inhibits the expression of a reporter gene. Thus, either increased or reduced expression of a reporter gene may be indicative of specific peptide binding to a target molecule.

The reporter gene may be any reporter gene known in the art. In certain embodiments, the reporter gene may encode a DNA modifying enzyme. That is, expression of the DNA modifying enzyme may result in the modification of a target DNA sequence and the degree of modification can subsequently be determined by sequencing. Accordingly, the measurable output signal would be the degree of modification of the target DNA sequence. In certain embodiments, the target DNA sequence is encoded in the DNA library. That is, the degree of modification of the target DNA sequence and the identity of the peptide candidate that induced the modification may be determined in a single sequencing read.

In other embodiments, the reporter gene may be a selection marker. That is, specific binding of a peptide to a non-essential cell component may result in reduced cell viability under selective conditions. For example, the reporter gene may be an antibiotic resistance gene such that the host cell can only grow in the presence of the respective antibiotic when the reporter gene is expressed. Alternatively, the selection marker may be an auxotrophic marker such that the host cell can only grow in the absence of a respective nutrient when the reporter gene is expressed. Accordingly, specific binding of a peptide to a non-essential target molecule may also result in altered cell viability. For that, the target molecule has to be functionally and/or covalently linked to a recombinant biosensor, which in turn regulates the expression of a selection marker. In this case, the measurable output signal may be the abundance of next generation sequencing reads in a sample that correspond to a particular library member

Accordingly, in a particular embodiment, the invention relates to the method according to the invention, wherein the reporter gene is (i) an antibiotic resistance gene; (ii) an auxotrophic marker; or (iii) a gene encoding a DNA-modifying enzyme.

In a particular embodiment, the invention relates to the method according to the invention, wherein the peptide candidate or the peptide synthesized by the candidate complex of non-ribosomal peptide biosynthesis enzymes is a linear, a cyclic or a branched peptide.

The present invention relates to a method for screening DNA libraries encoding a plurality of peptides. Preferably, the DNA library comprises a plurality of circular DNA molecules or vectors, such as, without limitation plasmids, cosmids, bacterial artificial chromosomes (BAC), yeast artificial chromosomes (YAC) and vectors derived from bacteriophages or plant or animal (including human) viruses. In certain embodiments, peptides are directly encoded in the library. That is, the library may comprise a plurality of nucleic acid sequences that can be directly transcribed and translated into peptides. Alternatively, peptides may be non-ribosomal peptides that are synthesized enzymatically. In such embodiments, the DNA library encodes a plurality of enzymes or enzyme complexes that catalyze the synthesis of non-ribosomal peptides. Preferably, every member of a DNA library encodes a single peptide or a single complex of non-ribosomal peptide biosynthesis enzymes.

When a peptide is directly encoded in a DNA library, the peptide is usually translated into a linear peptide. However, such ribosomal peptides may be circularized post-translationally by appropriate enzymes. Enzymes that catalyze the circularization of peptides are known in the art and the skilled person is aware of methods to introduce such enzymes into a host cell.

In the case of non-ribosomal peptides, it is to be understood that the non-ribosomal peptides may be linear, circular or branched. The term "branched peptide" is inclusive of any peptide having at least one amino acid covalently attached to a side-group of another amino acid. Circular non-ribosomal peptides may be circularized by an enzyme that is comprised in the complex of non-ribosomal peptide biosynthesis enzymes.

Within the present invention, a circular peptide may be a "head-to-tail" cyclic peptide, wherein the amino acid at the N-terminus of the peptide forms a covalent bond with the amino acid at the C-terminus of the peptide. In certain embodiments, cyclic peptides comprise a peptide bond between the amino group of the N-terminal amino acid and the carboxyl group of the C-terminal amino acid. In other embodiments, a circular peptide may be a peptide wherein the N- or C-terminal amino acid of the peptide forms a covalent bond with an internal amino acid. In such embodiments, the circular peptide is at the same time a branched peptide.

In certain embodiments, the DNA library may encode exclusively linear peptides. In certain embodiments, the DNA library may encode exclusively circular peptides. In certain embodiments, the DNA library may encode a mixture of linear, circular and/or branched peptides.

In a particular embodiment, the invention relates to the method according to the invention, wherein the DNA library encodes at least one peptide candidate and at least one candidate complex of non-ribosomal peptide biosynthesis enzymes.

In certain embodiments, the DNA library may exclusively encode linear ribosomal peptides, preferably wherein each member of the DNA library encodes a single ribosomal peptide. In other embodiments, the DNA library may exclusively encode complexes of non-ribosomal peptide biosynthesis enzymes, preferably wherein each member of the DNA library encodes a single complex of non-ribosomal peptide biosynthesis enzymes.

However, the DNA library may also be a mixed library that encodes both ribosomal peptides and non-ribosomal peptides. Accordingly, the method of the invention may be used to simultaneously identify target-binding ribosomal and non-ribosomal peptides. The skilled person is aware of methods to obtain libraries encoding ribosomal peptides and non-ribosomal peptides. Such libraries may be obtained, for example, by mixing a DNA library encoding ribosomal peptides with a library encoding complexes of non-ribosomal peptide biosynthesis enzymes. Alternatively, a mixture of nucleic acid fragments encoding ribosomal peptides and nucleic acid fragments encoding complexes of non-ribosomal peptide biosynthesis enzymes may be cloned into an expression vector.

In a particular embodiment, the invention relates to the method according to the invention, wherein the host cell is a microbial host cell, in particular wherein the microbial host cell is a yeast cell or a bacterial cell.

In certain embodiments, the host cell is a microbial host cell. That is, in certain embodiments, the host cell may be a bacterial cell or a yeast cell. Preferably, the host cell is a host cell that can be efficiently transformed, transfected or transduced with a DNA library. In certain embodiments, the host cell is *Escherichia coli.* In certain embodiments, the host cell is *Saccharomyces cerevisiae.*

In certain embodiments, the method of the invention may be used to identify antimicrobial peptides directly in a pathogenic organism. By screening a DNA library directly in a pathogenic organism, peptides may be identified that specifically inhibit an essential component of the specific pathogen.

In certain embodiments, the pathogen may be a pathogenic bacterium. In certain embodiments, the pathogen may be *Pseudomonas aeruginosa.* In certain embodiments, the pathogen may be *Klebsiella pneumonia.* In certain embodiments, the pathogen may be *Acinetobacter* spp.. In certain embodiments, the pathogen may be *Escherichia coli.* In certain embodiments, the pathogen may be *Enterobacter* sp. In certain embodiments, the pathogen may be *Staphylococcus aureus.* In certain embodiments, the pathogen may be *Enterococcus* sp.

Accordingly, in a particular embodiment, the invention relates to the method according to the invention, wherein the bacterial cell is a pathogenic bacterium, in particular wherein the pathogenic bacterium is selected from the group consisting of: *Pseudomonas aeruginosa, Klebsiella pneumonia, Acinetobacter* spp., *Escherichia coli, Enterobacter* sp., *Staphylococcus aureus* and *Enterococcus* sp.

In a particular embodiment, the invention relates to the method according to the invention, wherein the host cell is a protease deficient host cell.

To improve the stability of ribosomal and/or non-ribosomal peptides in the host cell, the host cell may be a protease deficient host cell. The term "protease deficient host cell" as used herein refers to a host cell in which at least one endogenous protease or peptidase is inhibited, down-regulated and/or knocked out. Preferably, one or more genes encoding endogenous proteases and/or peptidases are knocked out in the host cells. In *Escherichia coli* preferably at least one of the genes *abgA, ampA, ampM, clpP, dacA, dacB, dacC, dacD, dcp, ddpX, degP, degQ, degS, elaD, frvX, ftsH, gcp, glpG, hchA, hflB, hofD, hopD, hsIV, htpX, htrA, hyaD, hybD, hycH, hycl, iadA, iap, IdcA, IepB, Ion, IspA, mepA, nlpC, nlpD, ompT, opdA, opdB, pbpG, pepD, pepE, pepP, pepQ, pepT, pmbA, pppA, pqqL, prlC, ptrA, ptrB, sohB, sppA, sprT, tesA, tldD, tldE, tsp, umuD, vanX, YaeL, yafL, ycaL, ycbZ, ydcP, ydgD, ydhO, yeaZ, yebA, yegQ, yfbL, ygeY, yggG, yhbO, yhbU, yhjJ, yibG, ypdF is* knocked out or down-regulated. Genes encoding proteases or peptidases may be knocked out or down-regulated by any method known in the art.

In a particular embodiment, the invention relates to the method according to the invention, wherein the host cell comprises an enzyme that catalyses the cyclization of a candidate peptide.

That is, the host cell may further comprise at least one enzyme that catalyzes the cyclization of candidate peptides. Preferably, the enzyme that catalyses the cyclization of candidate peptides is a peptide ligase, as summarized by Nuijens et al. (Natural Occurring and Engineered Enzymes for Peptide Ligation and Cyclization. Front. Chem. 7:829. doi: 10.3389/fchem.2019.00829). Other enzymes that may be used for the cyclization of peptides include macrocyclases, peptide cyclases, peptide ligases and split-inteins.

In a particular embodiment, the invention relates to the method according to the invention, wherein the abundance of measurable output signals in a sample is determined by sequencing, in particular by next-generation sequencing.

Within the present invention, the determination whether a peptide is a target-binding peptide is made based on the abundance of measurable output signals. The method for quantifying measurable output signals depends to a large extent on the nature of the signal.

Within the present invention, it is preferred that the measurable output signal is quantified by sequencing, more preferably by next generation sequence. In certain embodiments, the measurable output signal corresponds to cell viability. For example, peptides that directly bind to and inhibit an essential component of a host cell will eventually reduce the cell viability of the host cell. This reduced cell viability will result in a lower proportion of host cells in a sample encoding said peptide and consequently in a lower abundance of next-generation sequencing reads for said peptide.

Accordingly, expression of a target-binding peptide will result in a lower abundance of measurable output signals for said peptide after the induction step.

When the target molecule is functionally linked to a recombinant biosensor, specific binding of a peptide to the target molecule may induce or inhibit in the expression of a selection marker, depending on the configuration of the reporter system. Thus, specific binding of a peptide to a target molecule may either reduce or maintain cell viability under selective conditions. In embodiments where specific binding of a peptide to a target molecule reduces cell viability, a lower abundance of next-generation sequencing reads will be obtained for said peptide, since host cells expressing this peptide will be diluted out of the culture. In embodiments where specific binding of a peptide to a target molecule maintains cell viability, a higher abundance of next-generation sequencing reads will be obtained for said peptide, since only cells that express a target-binding peptide are able to propagate under selective conditions. Accordingly, expression of a target-binding peptide may result in a lower or higher abundance of measurable output signals after the induction step, depending mainly on the configuration of the reporter system.

Another measurable output signal that may be quantified by next-generation sequencing is the degree of DNA modifications. In certain embodiments, the expression of a reporter gene encoding a DNA modification enzyme is controlled by a recombinant biosensor. Specific binding of a peptide to a target molecule may either induce or inhibit the expression of the DNA modification enzyme. In such embodiments, the host cell comprises a target DNA sequence which can be modified by the DNA modification enzyme. The degree of DNA modification in the target DNA sequencing can then be used as a measurable output signal to identify a target-binding peptide. Again, depending on the configuration of the reporter system, specific binding of a peptide to a target molecule may either result in increased or decreased modification of the target DNA sequence.

The term "next generation sequencing", as used herein, refers to sequencing technologies having increased throughput as compared to traditional Sanger- and capillary electrophoresis-based approaches, for example with the ability to generate hundreds of thousands or millions of relatively short sequence reads at a time. Some examples of next generation sequencing techniques include, but are not limited to, sequencing by synthesis, sequencing by ligation, and sequencing by hybridization. Examples of next generations sequencing methods include pyrosequencing as used by the GS Junior and GS FLX Systems (454 Life Sciences), sequencing by synthesis as used by Illumina's Miseq and Solexa system, the SOLiD™ (Sequencing by Oligonucleotide Ligation and Detection) system (Life Technologies Inc.), and Ion Torrent Sequencing systems such as the Personal Genome Machine or the Proton Sequencer (Life Technologies Inc), and nanopore sequencing systems (Oxford nanopore). Alternatively, the sequencing method may Single Molecule, Real-Time (SMRT) Sequencing as established by Pacific Biosciences. That is, the term "next-generation sequencing" also comprises "third-generation sequencing" methods.

In a particular embodiment, the invention relates to the method according to the invention, the method comprising an additional step of overexpressing a target molecule in the plurality of host cells.

Within the present invention, the target molecule is either an endogenous essential component of a host cell or a non-essential cell component that is comprised in or functionally linked to a recombinant biosensor. When aiming to identify peptides that bind to a target molecule with very high affinity, the host cell may be desensitized or hyposensitized by overexpressing the target molecule. That is, the level of target molecule in a host cell may be artificially increased such that either higher amounts of peptides or more efficiently binding peptides will be required to obtain significant changes in the abundance of the measurable output signal. Since the amount of peptide is held constant by the expression system of the DNA library, overexpressing a target molecule may help identifying peptides that bind to the target molecule with very high affinity.

In certain embodiments, the target molecule is a protein or a peptide. In such embodiments, the target molecule may be overexpressed in the host cell by methods well known in the art. In certain embodiments, the entire target molecule may be overexpressed in the host cell. However, in certain embodiments, it will be sufficient to overexpress only the fragment of the target molecule that is specifically bound by a peptide. For example, when the target molecule is an enzyme and the method of the invention is employed to identify peptides that inhibit the enzyme, only a fragment of the enzyme comprising the active site may be overexpressed.

In certain embodiments, the target molecule may be an endogenous protein of the host cell. In such embodiments, the host cell may be hyposensitized by overexpressing the target molecule, or a fragment thereof, in the host cell from a plasmid.

In certain embodiments, two variants of a target molecule may be expressed. That is, a target molecule, or a fragment thereof, may be expressed as part of a recombinant biosensor. In addition, the target molecule, or a fragment thereof, may be separately expressed, independent of the biosensor. In such embodiments, the separately expressed target molecule will neutralize at least part of the peptides in the cell, such that only peptides with an exceptionally high affinity can bind to the target molecule comprised in the biosensor and thereby activate the biosensor.

It is important to understand that a target molecule may be directly or indirectly overexpressed. That is, in certain embodiments, the target molecule may be an RNA, a peptide or a protein. In such embodiments, the target molecule may be directly overexpressed in the host cells. However, in certain embodiments, the target molecule may be, without limitation, a lipid, a lipopolysaccharide or a polysaccharide. In such embodiments, the level of the target molecule may be increased in the host cell by overexpressing enzymes that are involved in the biosynthesis of these molecules. Thus, the term "overexpression of a target molecule" also includes the overexpression of enzymes that are involved in the biosynthesis of non-protein and non-nucleic acid target molecules, even if these enzymes may not be the direct target of a peptide candidate.

In a particular embodiment, the invention relates to the method according to the invention, wherein the DNA library comprises a nucleic acid molecule encoding a target molecule, preferably wherein each member of the DNA library encodes (i) a peptide candidate or a candidate complex of complex of non-ribosomal peptide biosynthesis enzymes; and (ii) a target molecule.

That is, the additional target molecule is preferably encoded in the DNA library. Preferably, each member of the DNA library encodes a single peptide or a single complex of non-ribosomal peptide biosynthesis enzymes and a target molecule.

In certain embodiments, a DNA library may encode a plurality of peptides and/or complexes of non-ribosomal peptide biosynthesis enzymes and a single target molecule. Such libraries may be used for hypo-sensitization of the host cell and to identify peptides that bind to the target molecule encoded in the DNA library with very high affinity.

Alternatively, a DNA library may encode a single peptide or a single complex of non-ribosomal peptide biosynthesis enzymes and a plurality of target molecules. Such libraries may be used to identify the target molecule of the peptide encoded in the DNA library.

Alternatively, a DNA library may encode a plurality of peptides and/or complexes of non-ribosomal peptide biosynthesis enzymes and a plurality of target molecules. Such libraries may be used to identify novel target-binding peptides and, in the same experiment, identify the respective target molecules of at least some of these peptides.

In a particular embodiment, the invention relates to the method according to the invention, the method comprising an additional step of expressing a small inhibitory RNA in the plurality of host cells, in particular wherein the small inhibitory RNA inhibits the synthesis of a target molecule.

Instead of hypo-sensitizing the host cell by overexpressing a target molecule, the host cell may also be hyper-sensitized. Hyper-sensitization of host cells is preferably achieved by overexpressing a small inhibitory RNA that inhibits the synthesis of a particular target molecule. Hyper-sensitization may, for example, be applied when attempting to identify peptides that bind to target molecules that are considered to be undruggable. By inhibiting the synthesis of a target molecule with an inhibitory small RNA, the level of said target molecule in the host cell may be reduced to a level that is just sufficient to support survival of the host cell. In such embodiments, even weak binding peptides may be sufficient to neutralize the remaining target molecules and thereby result in a significant change in the abundance of a measurable output signal.

Inhibiting the synthesis of target molecules via small inhibitory RNAs is preferably applied in embodiments where the target molecules are essential endogenous cell components, in particular essential endogenous proteins. In such embodiments, the levels of one or more essential host protein may be downregulated by small inhibitory RNAs such that the remaining essential host protein can be inhibited even by peptides that bind to the target molecule (the essential host protein) with low affinity.

It is important to understand that the inhibitory RNA may directly or indirectly inhibit the synthesis of a target molecule. That is, in embodiments where the target molecule is a peptide or a protein, the inhibitory RNA may directly interfere with the synthesis of said peptide or protein. In embodiments where the target molecule is, without limitation, a lipid, a lipopolysaccharide or a polysaccharide, the inhibitory RNA may indirectly interfere with the synthesis of said target molecules by inhibiting the synthesis of an enzyme that is involved in the biosynthesis of these target molecules.

In a particular embodiment, the invention relates to the method according to the invention, wherein the DNA library comprises a nucleic acid molecule encoding a small inhibitory RNA, preferably wherein each member of the DNA library encodes (i) a peptide candidate or a candidate complex of complex of non-ribosomal peptide biosynthesis enzymes; and (ii) a small inhibitory RNA.

That is, the small inhibitory RNA is preferably encoded in the DNA library. Preferably, each member of the DNA library encodes a single peptide or a single complex of non-ribosomal peptide biosynthesis enzymes and a small inhibitory RNA.

In certain embodiments, a DNA library may encode a plurality of peptides and/or complexes of non-ribosomal peptide biosynthesis enzymes and a single small inhibitory RNA. Such libraries may be used to hyper-sensitize a host cell in order to identify weak binding peptides, such as peptides that bind to a target molecule that was previously considered undruggable.

Alternatively, a DNA library may encode a single peptide or complex of non-ribosomal peptide biosynthesis enzymes and a plurality of small inhibitory RNAs. Such libraries may be used to identify the target molecule of the peptide encoded in the DNA library.

Alternatively, a DNA library may encode a plurality of peptides and/or complexes of non-ribosomal peptide biosynthesis enzymes and a plurality of small inhibitory RNAs. Such libraries may be used to identify novel target-binding peptides and, in the same experiment, identify the respective target molecules of at least some of these peptides.

In a particular embodiment, the invention relates to a DNA library comprising at least two plasmids, wherein each of the at least two plasmids comprises a nucleotide sequence encoding (a) a peptide candidate or a candidate complex of non-ribosomal peptide biosynthesis enzymes; and (b) a target molecule or an enzyme that is involved in the biosynthesis of a target molecule.

Certain embodiments of the invention relate to a DNA library that may be used in the method according to the invention. Preferably the DNA library comprises a plurality of DNA elements. More preferably, the DNA library comprises a plurality of circular DNA elements. Most preferably the DNA library comprises a plurality of plasmids.

At least two members of the DNA library may encode a peptide or a complex of non-ribosomal peptide biosynthesis enzymes. Besides peptides and/or complexes of non-ribosomal peptide biosynthesis enzymes, the DNA library of the invention may further encode at least one target molecule. That is, the DNA library according to the invention may be used for the screening of peptides in a hypo-sensitized cell.

In a particular embodiment, the invention relates to the DNA library according to the invention, wherein in each of the at least two plasmids, the nucleotide sequence encoding the peptide candidate or the candidate complex of non-ribosomal peptide biosynthesis enzymes is operably linked to a first promoter sequence and wherein the nucleotide sequence encoding the target molecule or the enzyme that is involved in the biosynthesis of a target molecule is operably linked to a second promoter sequence.

That is, the nucleotide sequence encoding the peptide or the complex of non-ribosomal peptide biosynthesis enzymes and the nucleotide sequence encoding the target molecule may be under the control of independent promoters.

Within the present invention, it is preferred that the nucleotide sequences encoding the peptides and/or the complexes of non-ribosomal peptide biosynthesis enzymes are under control of an inducible promoter. Accordingly, the synthesis of ribosomal and/or non-ribosomal peptides in the host cell can be switched on in the presence of an inducer molecule. The choice of promoter depends on the host cell. The skilled person is aware of various inducible promoters that may be used for a respective host cell.

The one or more target molecule may be expressed from a second promoter. The second promoter may be another inducible promoter or a constitutive promoter. Preferably, the function of the second promoter is independent from the function of the first promoter. That is, expression of the peptide/complex of non-ribosomal peptide biosynthesis enzymes and of the target molecule is preferably regulated separately.

In certain embodiments, expression of the peptide/complex of non-ribosomal peptide biosynthesis enzymes is driven from an inducible promoter and expression of the target molecule is driven from a constitutive promoter. In certain embodiments, both the expression of the peptide/complex of non-ribosomal peptide biosynthesis enzymes and of the target molecule is driven from independent inducible promoters.

In a particular embodiment, the invention relates to the DNA library according to the invention, wherein the target molecule is a protein originating from a microbial host cell, in particular from a pathogenic microbial host cell.

That is, the target molecule may be an endogenous protein of the host cell. Preferably, the target molecule may be an essential endogenous protein of the host cell. In certain embodiments, the target molecule may be an essential endogenous protein of a pathogenic host cell. Accordingly, the DNA library of the invention may be used for the identification of highly efficient anti-microbial peptides in a hypo-sensitized microbial host organism.

In a particular embodiment, the invention relates to the DNA library according to the invention, wherein the target molecule is a protein that is involved in a human disease.

Alternatively, the DNA library of the invention may be used for the identification of peptide drugs for the treatment and/or prevention of non-infectious diseases. Identification of such peptides requires an engineered host cell that reflects a human disease model involving the target molecule. In such embodiments, additional overexpression of the target molecule from the DNA library may lead to hypo-sensitization of the human disease model and may enable the identification of highly efficient peptides.

In a particular embodiment, the invention relates to a DNA library comprising at least two plasmids, wherein each of the at least two plasmids comprises a nucleotide sequence encoding (a) a peptide candidate or a candidate complex of non-ribosomal peptide biosynthesis enzymes; and (b) a small inhibitory RNA.

In other embodiments, the DNA library may encode one or more peptides/complexes of non-ribosomal peptide biosynthesis enzymes and one or more small inhibitory RNA. Preferably, the small inhibitory RNA inhibits the synthesis of a target molecule and thereby down-regulates the level of said target molecule in the host cell. That is, the DNA library according to the invention may be used for the screening of peptides in a hyper-sensitized host cell.

In a particular embodiment, the invention relates to the DNA library according to the invention, wherein in each of the at least two plasmids, the nucleotide sequence encoding the peptide candidate or the candidate complex of non-ribosomal peptide biosynthesis enzymes is operably linked to a first promoter sequence and wherein the nucleotide sequence encoding the small inhibitory RNA is operably linked to a second promoter sequence.

That is, the nucleotide sequence encoding the peptide or the complex of non-ribosomal peptide biosynthesis enzymes and the nucleotide sequence encoding the small RNA may be under the control of different promoters.

The small inhibitory RNA may be expressed from an inducible promoter or from a constitutive promoter. Preferably, the function of the promoter controlling expression of the small inhibitory RNA is independent from the function of the promoter controlling expression of the peptide/complex of non-ribosomal peptide biosynthesis enzymes. That is, expression of the peptide/complex of non-ribosomal peptide biosynthesis enzymes and of the small inhibitory RNA is regulated independently.

In certain embodiments, expression of the peptide/complex of non-ribosomal peptide biosynthesis enzymes is driven by an inducible promoter and expression of the small inhibitory RNA is driven by a constitutive promoter. In certain embodiments, both the expression of the peptide/complex of non-ribosomal peptide biosynthesis enzymes and of the small inhibitory RNA is driven by independent inducible promoters.

In a particular embodiment, the invention relates to the DNA library according to the invention, wherein the inhibitory small RNA inhibits synthesis of an essential protein from a microbial host organism, in particular from a pathogenic microbial host organism.

That is, the small inhibitory RNA may be used to lower the intracellular levels of an endogenous target molecule in the host cell. When the endogenous target molecule is an essential component of the host cell, lowering the intracellular levels of the target molecule with a small inhibitory RNA may allow identifying peptides that bind to the target molecule with only low affinity.

Within the present invention, a DNA library may encode (a) a plurality of peptides and/or complexes of non-ribosomal peptide biosynthesis enzymes and (b) a single target molecule or a single small inhibitory RNA. That is, a DNA library may encode at least 100, at least 500, at least 1'000, at least 2'000, at least 3'000, at least 4'000, at least 5'000, at least 6'000, at least 7'000, at least 8'000, at least 9'000, at least 10'000, at least 20'000, at least 50'000 or at least 100'000 peptides and/or complexes of non-ribosomal peptide biosynthesis enzymes. Such libraries may be used to identify peptides that specifically bind to the target molecule that is encoded in the library or that is targeted by the small inhibitory RNA encoded in the library.

Alternatively, a DNA library may encode (a) a single peptide or a single complex of non-ribosomal peptide biosynthesis enzymes and (b) a plurality of target molecules or small inhibitory RNAs. That is, a DNA library may encode at least 100, at least 500, at least 1'000, at least 2'000, at least 3'000, at least 4'000, at least 5'000, at least 6'000, at least 7'000, at least 8'000, at least 9'000, at least 10'000 or at least 20'000 target molecules or small inhibitory RNAs. Such libraries may be used to identify the target molecule of a specific peptide.

Alternatively, a DNA library may encode (a) a plurality of peptides and/or complexes of non-ribosomal peptide biosynthesis enzymes and (b) a plurality of target molecules or small inhibitory RNAs. That is, a DNA library may encode at least 100, at least 500, at least 1'000, at least 2'000, at least 3'000, at least 4'000, at least 5'000, at least 6'000, at least 7'000, at least 8'000, at least 9'000, at least 10'000, at least 20'000, at least 50'000 or at least 100'000 peptides and/or complexes of non-ribosomal peptide biosynthesis enzymes and at least 100, at least 500, at least 1'000, at least 2'000, at least 3'000, at least 4'000, at least 5'000, at least 6'000, at least 7'000, at least 8'000, at least 9'000, at least 10'000 or at least 20'000 target molecules or small inhibitory RNAs. Such libraries may be used to identify novel target-binding peptides and, in the same experiment, identify the respective target molecules of at least some of these peptides.

## BRIEF DESCRIPTION OF THE DRAWINGS

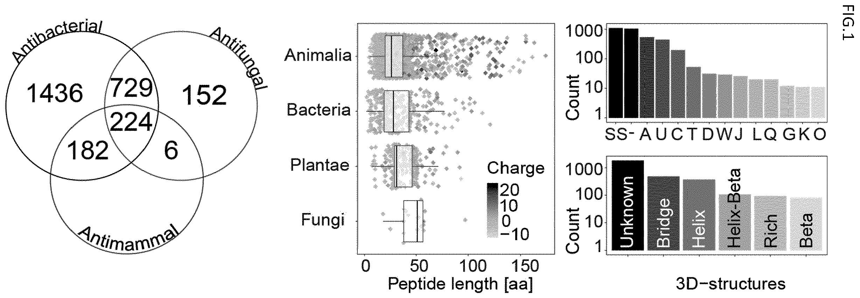

**FIG.1.** Biological diversity of PARENTS. PARENTS are derived from the AMP database (APD). They have experimentally proven biological activity, e.g. antibacterial (Gram-negative and/or Gram-positive bacteria), antifungal, or antimammal (hemolytic or anticancer). They originate from species of various kingdoms of life and differ considerably by length, charge, chemical modification (among others: SS = disulfide bridges, A = amidation, U = terminal Rana box, C = backbone cyclization, T = thioether bridges, D = d-amino acids, W = dehydration, J = sidechain cyclization, L = lipidation, Q = terminal glutamate, E = acetylation, G = glycosylation, K = hydroxylation, - = no modification reported), and 3D-structure (Beta = beta-sheet, Bridge = disulfide bond, Helix = alpha-helix, Helix-Beta = alpha-helix and beta-sheet, Rich = rich in unusual amino acids, Unknown = no reported structure).

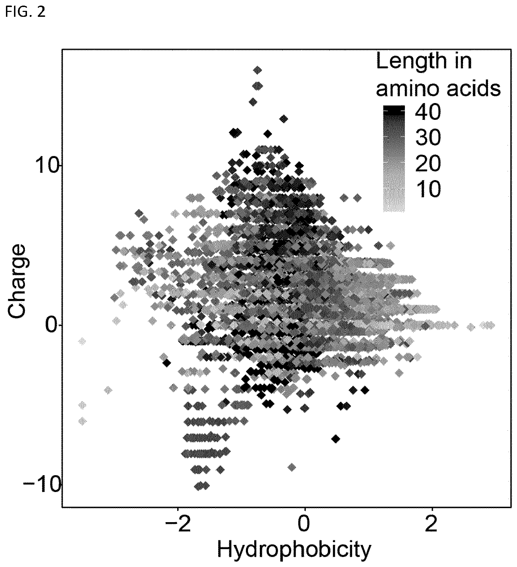

**FIG.2.** Predicted physiochemical properties of peptides in the library. All peptides of the library are plotted according to their charge and hydrophobicity at pH 7 and shaded by their length. Mean charge = +2.3; mean hydrophobicity (GRAVY scale) = 0.0; mean length = 27 amino acids.

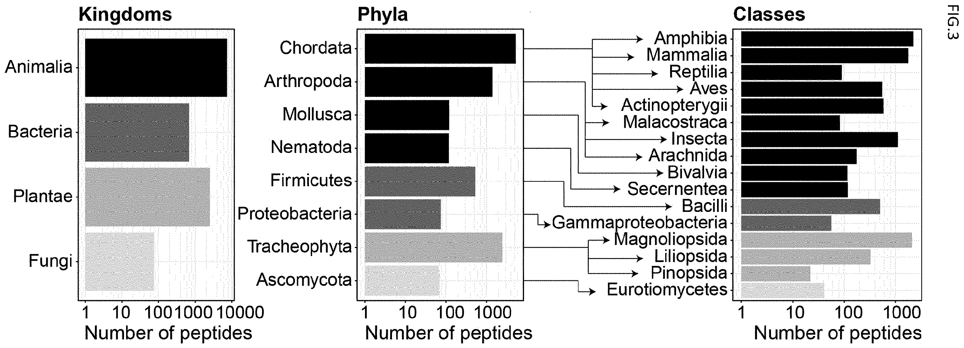

**FIG.3.** Taxonomical classification of the peptide library. All peptides of the library are grouped by their taxa in rank kingdom, phylum, and class of the host from which their sequences had been derived. Only groups comprised of at least 20 peptides are displayed. Phyla and classes are shaded by their kingdom (left).

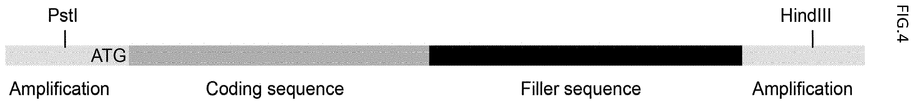

**FIG.4.** Sequence architecture of peptide-encoding DNA sequences as used for synthesis. Each DNA sequence contains a peptide coding sequence, a unique filler sequence used for standardizing sequence length to 170 nucleotides, and two universal amplification sites used for both, cloning and amplification. The coding sequences are generated by reverse translation of the respective peptide amino acid sequence followed by codon optimization for expression in *E. coli.* Amplification sites at the 5' and 3' end are the same for all inserts and contain restriction sites for subsequent integration into the multiple cloning site of the expression plasmid.

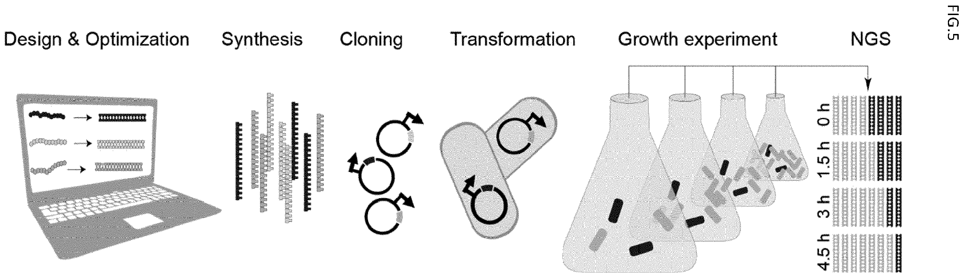

**FIG.5.** Experimental workflow: Design & Optimization: Peptide sequences are reverse translated into *E. coli* codon-optimized nucleotide sequences. Synthesis: All peptide-encoding sequences are synthesized as oligonucleotides. Cloning: The sequences are inserted into plasmids. Transformation: *E. coli* TOP10 is transformed with the generated peptide-encoding DNA library. Growth: Strains are incubated in shaking flasks, peptide expression is induced and plasmids are isolated. NGS: peptide-encoding DNA sequences are counted at four time points using NGS.

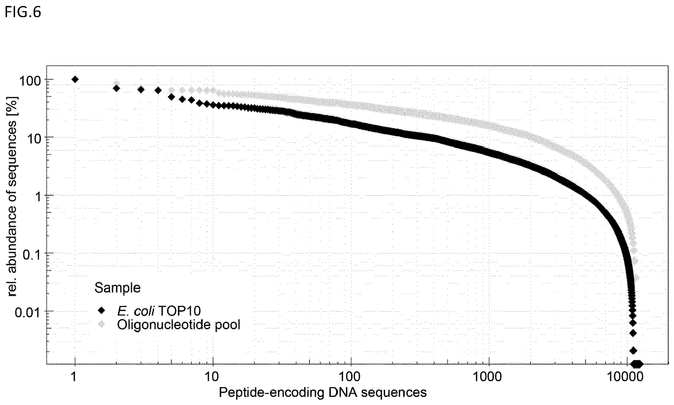

**FIG.6.** Relative abundance of peptide-encoding DNA sequences before and after cloning. Peptide-encoding DNA sequences in the original synthesized oligonucleotide pool (grey) and after insertion into a plasmid and transformation of *E. coli* TOP10, as used for the growth experiment (black) are counted by NGS. All counts are relative to the most abundant peptide-encoding sequence Chensinin-1CEb$_{2720\,NCBI}$, which appears 2,720 times (oligonucleotide pool) and 5,466 times (growth experiment), respectively.

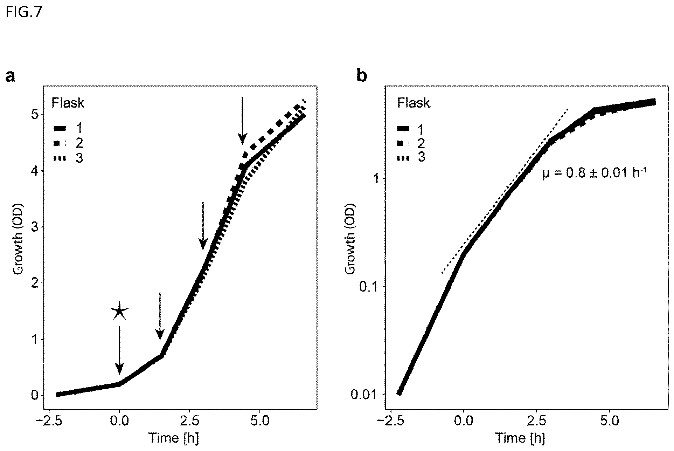

**FIG.7.** Growth of *E. coli* TOP10 expressing the peptide-encoded DNA library. a) Optical density at 600 nm (OD) is recorded over 8 h. Three 1-liter shake flasks containing 100 ml of LB-medium each are inoculated with 500 million cells of *E. coli* TOP10 carrying the peptide-encoded DNA library at -2.5 h (time reported relative to the time of induction). Peptides are expressed after 4 generations (0.0 h; OD~0.2) by adding $_L$-arabinose (asterisk). Cell samples for NGS are isolated from each replicate at the time of induction, and 1.5 h, 3.0 h, and 4.5 h post-induction (arrows). b) Log10 transformed data of (a) for calculation of the specific growth rate ($\mu$).

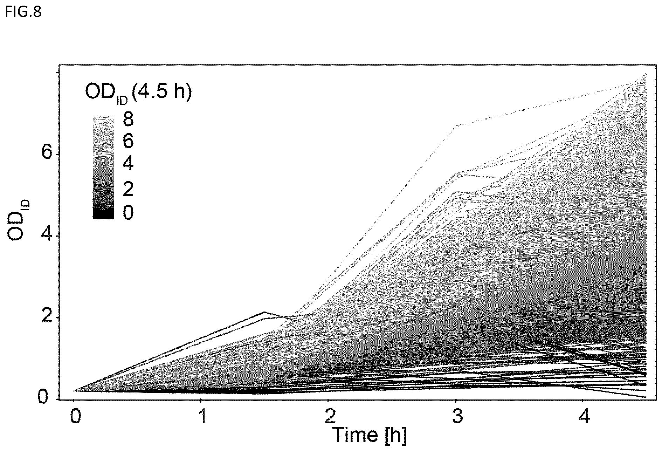

**FIG.8.** Growth curves of all 10,663 peptide-expressing strains, expressed as OD for a specific peptide-expressing strain (OD$_{ID}$; average of n=3). Shading from grey to black indicates higher growth inhibitory effects based on OD$_{ID}$ of last sampling point. Curves reaching a higher OD$_{ID}$ than eight (0.7 %) are omitted for clarity.

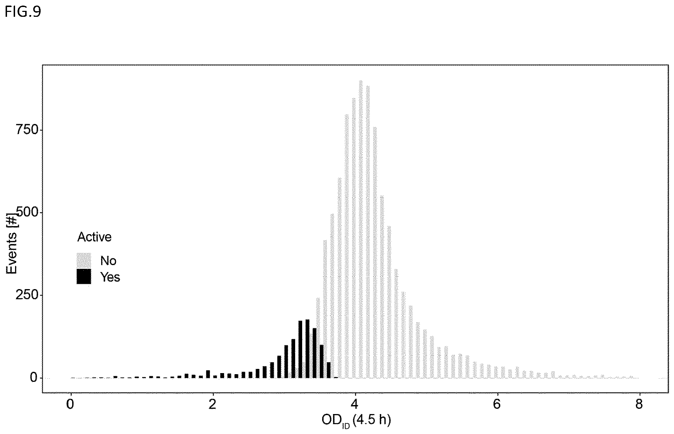

**FIG.9.** Growth inhibition as determined by OD$_{ID}$ at 4.5 h recorded for 10,663 peptide-expressing strains. OD$_{ID}$-values are recorded for each of the peptide-expressing strains and are averaged from the three replicates. Method-active peptides (black) significantly (Wald's test, adj. $p<0.05$) reduce the OD$_{ID}$ of their expressing strain after 4.5 h while method-inactive peptides (grey) fail to do so. Note that some candidates may also fail to reach statistical significance in the performed method due to low NGS read counts or high variance between replicates.

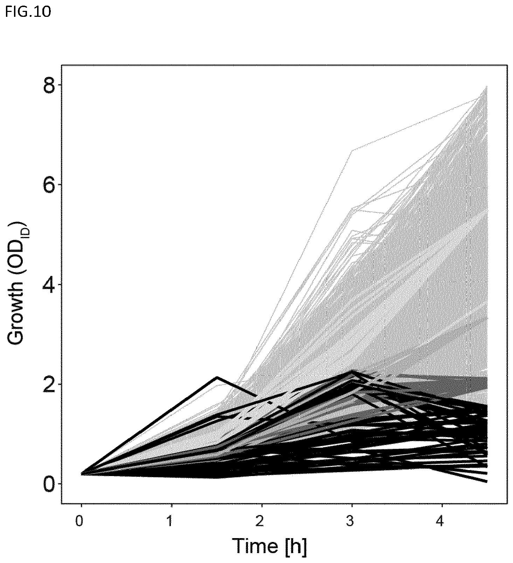

**FIG.10.** Growth of peptide-expressing *E. coli* strains in the method of the invention and in monoseptic cultures. a) All peptide-expressing strains are ranked by their growth inhibition after 4.5 h (= ranked by their OD$_{ID}$ (4.5 h)) and selected representatives were then subdivided into four groups: rank 1-50 (average OD$_{ID}$ (4.5 h) = 1.0); rank 100-119 (average OD$_{ID}$ (4.5 h) = 2.0); rank 1,000-1,019 (average OD$_{ID}$ (4.5 h) = 3.3); rank 10,000-10,019 (average OD$_{ID}$ (4.5 h) = 5.4) and HNP-$_{13425\,APD}$ (negative control, rank 2172, OD$_{ID}$ (4.5 h) = 3.7).

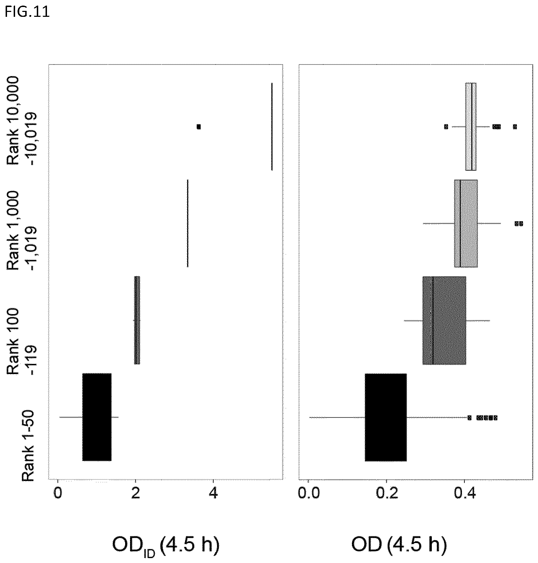

**FIG.11.** Boxplot of OD$_{ID}$ (4.5 h) and OD (4.5h) of the peptide-expressing strains in the different subgroups identified in (a). Note the different scales on the x-axis. Rank 1-50: average OD (4.5h) = 0.2. Rank 100-110: average OD (4.5h) = 0.35. Rank 1,000-1,019: average OD (4.5h) = 0.4. Rank 10,000-10,019: average OD (4.5h) = 0.42.

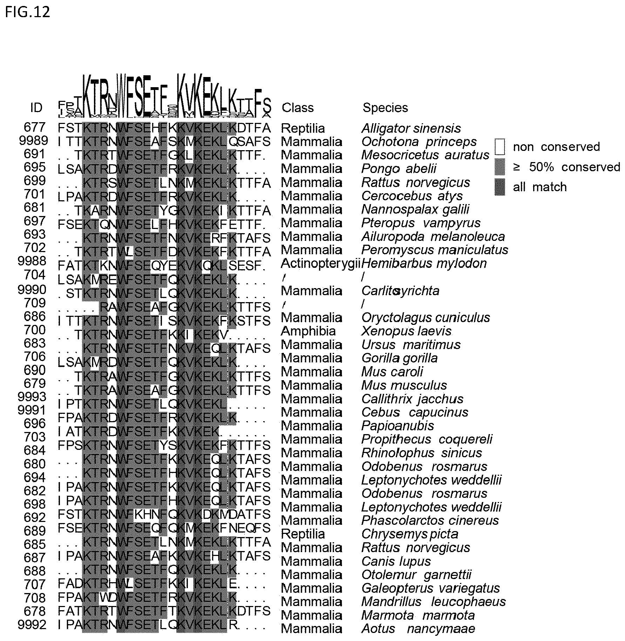

**FIG.12.** Analysis of Apo5 APOC1-derived $_{SIMILARS}$. a) Amino acid sequence alignment of all 36 SIMILARS of the Apo5 APOC1$_{677\,APD}$ PARENT. The inactive PARENT, derived from Chinese alligator (*Alligator sinensis*), and the only method-active similar (Apo5 APOC1$_{9989\,NCBI}$) derived from American pika (*Ochotona princeps*) differ by nine amino acids. The top shows the consensus sequence plot.

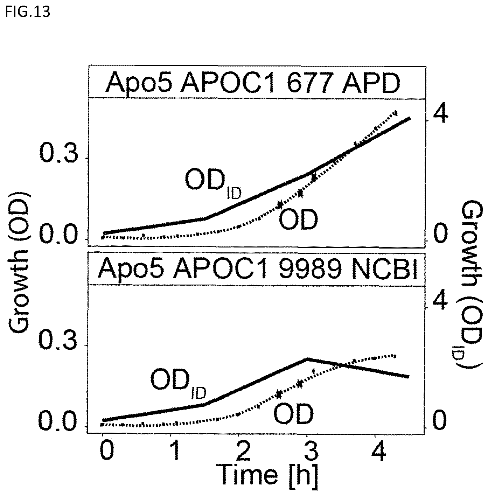

**FIG.13.** Overlay of growth curves recorded by the method of the invention (line, an average of n=3) and via monoseptic growth in microtiter plates (dotted line; n=3, error bars = 2 standard deviations), of *E. coli* TOP10 cells expressing Apo5 APOC1$_{677\,APD}$ and Apo5 APOC1$_{9989}$ NCBI.

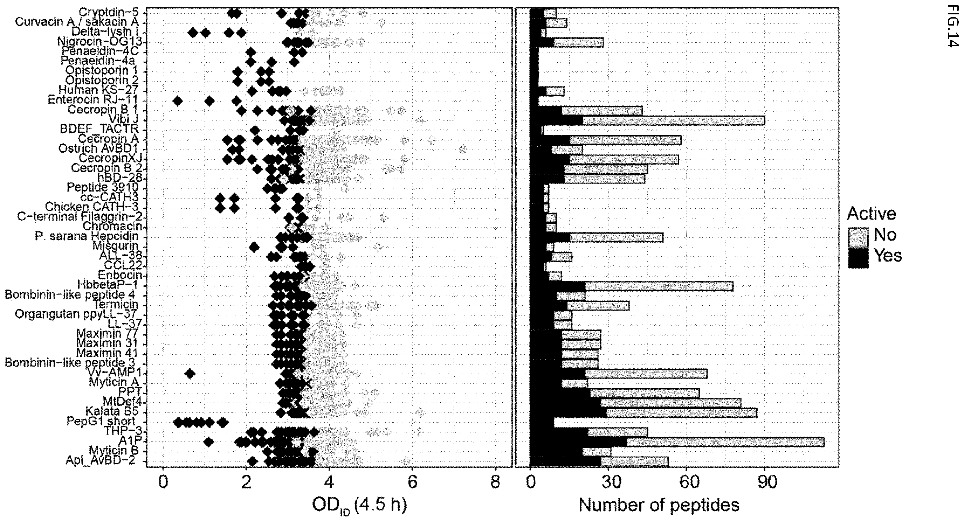

**FIG.14.** Overrepresentation of active $_{SIMILARS}$ derived from 47 $_{PARENTS}$. For 47 PARENTS (names on the left), method-actives were significantly overrepresented among the $_{SIMILARS}$ identified in the similarity search (Fisher's exact test, adj. $p<0.05$). The OD$_{ID}$ (4.5 h) values for the individual peptide-expressing strains within a group of PARENTS and $_{SIMILARS}$ are shown as dots (left) and the total number of active and non-active peptides for each of the 47 parents as bars (right).

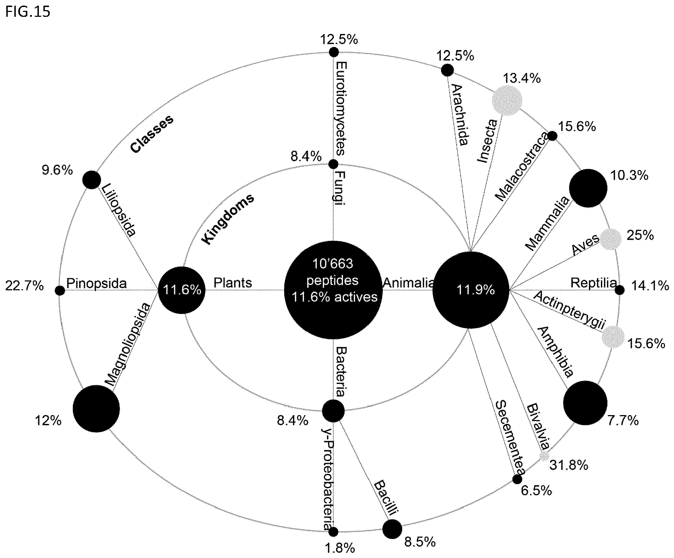

**FIG.15.** Taxa-specific method-activity in rank kingdom and class. All peptides tested are clustered taxonomically according to the host from which they were derived. The percentage of method-actives in the cluster is written next to (or inside) each circle. Compared to the 11.6% method-actives in the entire library, taxa in which method-actives are overrepresented (grey) are highlighted ($p<0.05$). Only clusters with more than 20 peptides are displayed. The circle area is representative of the total number of peptides within each cluster.

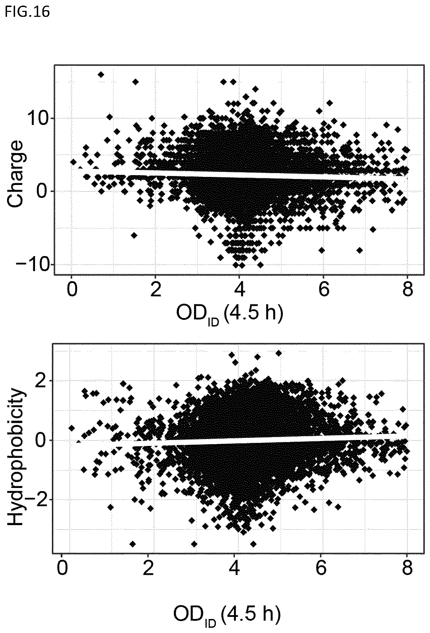

**FIG.16.** Influence of the physicochemical properties of peptides on method-activity. a) Charge (top) and hydrophobicity (bottom) of each peptide are plotted against $OD_{ID}$ (4.5h). Linear fits (both $p<0.01$, $R^2<0.001$, 10,661 DF) are displayed for the entire peptide library (white line).

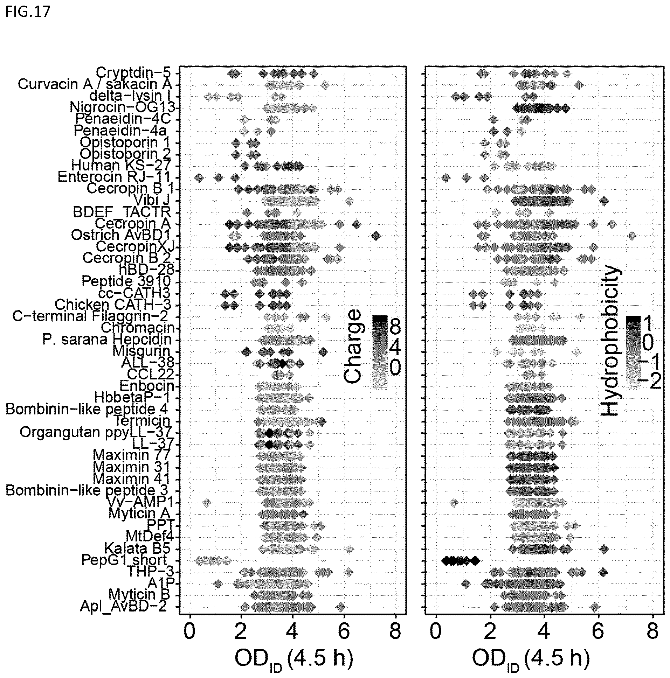

**FIG.17.** Charge (left) and hydrophobicity (right) are displayed for the 47 groups of PARENTS and their $_{SIMILARS}$ containing an overrepresentation of method-positives (Fisher's exact test, adj. $p<0.05$).

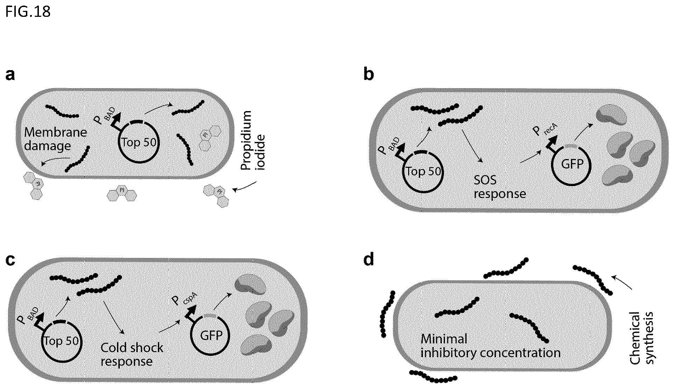

**FIG.18.** Assays to characterize the antimicrobial effect. (a-c) The 50 most method-active peptides (ranks 1-50; by $OD_{ID}$ (4.5 h)) are expressed intracellularly in a strain of *E. coli* TOP10 with or without a plasmid that expresses the gene for a reporter protein (green fluorescent protein, *gfg*) under the control of a promoter whose activity has been linked before to a specific stress response. a) Analysis of membrane damage by quantification of propidium iodide (PI) uptake. b-c) Quantification of GFP indicating the interference with intracellular targets by eliciting an SOS stress response indicative of DNA damage (readout via $P_{recA}$ (promoter of *E. coli's recA* gene)) or a cold shock response indicative of translation inhibition (readout via $P_{cspA}$ (promoter E. coli's *cspA* gene)). d) Method to determine the minimal inhibitory concentration (MIC). The 20 most active peptides are synthesized chemically, purified, and added to cultures of *E. coli* TOP10 and other pathogens.

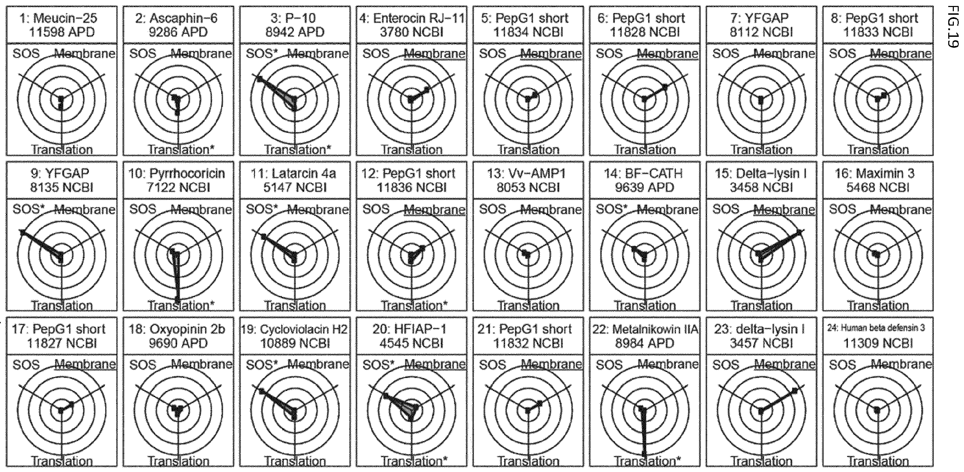

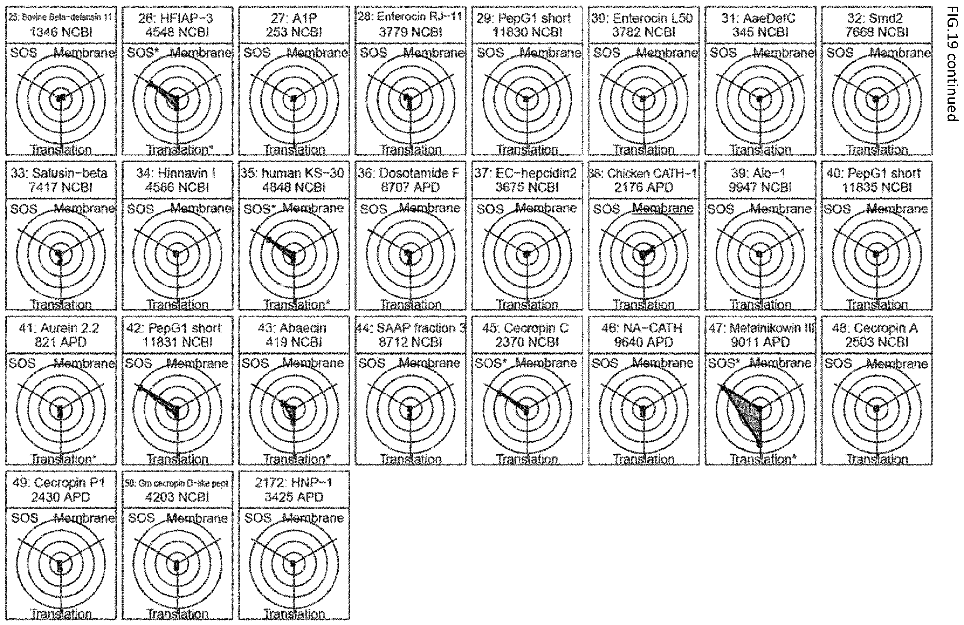

**FIG.19.** Characterization of the 50 most method-active peptides: Potential mechanisms of action. Each radar plot shows the mean SOS-response (DNA; activation of the *recA* promoter; n=3), translation inhibition (Translation; activation of the *cspA* promoter; n=3) and membrane-damage (Membrane; PI stained cells in percent; n=2) obtained after peptide expression in *E. coli* TOP10. Only the maximum and minimum values are reported in digits. The center represents values measured for the negative control peptide HNP-1$_{3425\,APD}$. Lower values are scaled to the center. Membrane damage is attributed if more than 10% of cells were PI-positive (underlined). For SOS and Translation, signals are reported relative to the signal obtained for the inactive control peptide HNP-1$_{3425\,APD}$. A significant increase (one-sided t-test, adj. $p<0.05$) compared to the inactive control is indicated by an asterisk (*).

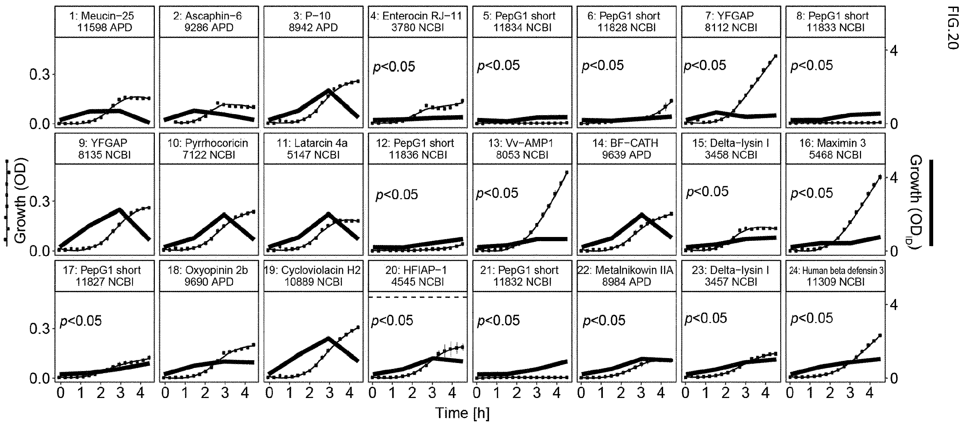

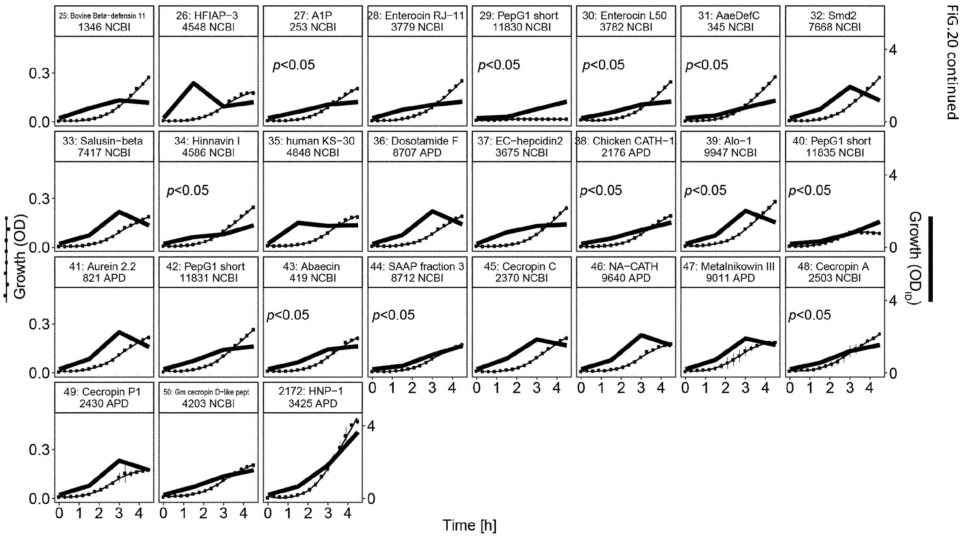

**FIG.20.** Characterization of the 50 most method-active peptides: Growth-curves. Black lines are recorded growth curves with the claimed method (average of n=3) determined via $OD_{ID}$ approximation (header: 'rank: parent name'). Black dotted lines are growth curves (n=3, error bars: 4 standard deviations) determined via OD measurement in microtiter plates of individually grown strains. In each facet, we state if we obtain a p<0.05 (Wald's test) for significant growth inhibition after 1.5 h.

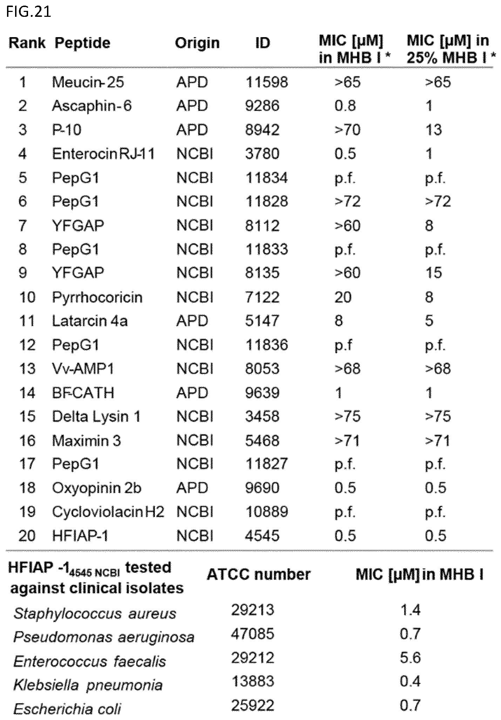

**FIG.21.** Antimicrobial activity in clinically relevant assays. Mean MIC-values are recorded (n=3) in microtiter plate assays using chemically synthesized peptides. (*) = against the screening strain *E. coli* TOP10; p.f. = purification failed.

## DETAILED DESCRIPTION

Certain aspects of the invention will be described in more detail in the following sections.

## 1. The target molecule

The present invention is directed to a method for identifying peptides that specifically bind to a target molecule. In its broadest meaning, the target molecule may be any molecule that can be specifically bound by a peptide. Preferably, the target molecule is a component of a cell. That is, the target molecule may be a component of a host cell (an endogenous target molecule) or a component of another cell (a heterologous target molecule). In certain embodiments, the target molecule is a peptide or a protein. In other embodiments, the target molecule may be a nucleic acid, such as a DNA or an RNA molecule. In further embodiments, the target molecule may be a lipid, such as, without limitation, lipid II, or a lipopolysaccharide (LPS), or any precursor thereof. Alternatively, the target molecule may be an enzyme that is involved in lipid biosynthesis.

In certain embodiments, the target molecule may be involved in a disease mechanism. That is, the target molecule may be a molecule comprised in a pathogenic organism or may be a molecule that is involved in the manifestation and/or progression of a disease.

The term "specific binding" as used herein, such as to describe interactions between a target molecule and a peptide, refers to the generally reversible binding of a peptide to a target molecule through the combined effects of spatial complementarity of target molecule and peptide structures coupled with electrostatic forces, hydrogen bonding, hydrophobic forces, and/or van der Waals forces between the two molecules. Generally, the greater the spatial complementarity and the stronger the other forces between two molecules, the greater will be the binding specificity of a peptide for a respective target molecule. Within the present invention, a peptide is said to specifically bind to a target molecule, if binding of the peptide to a target molecule induces a change in the abundance of a measurable output signals.

### 1.1 Endogenous target molecules

In certain embodiments, the target molecule may be an endogenous component of a host cell. The term "endogenous", as used herein, refers to any polynucleotide or polypeptide which is present and/or naturally expressed within a host cell. Preferably, the endogenous component is an essential component of the host cell. That is, the target molecule may be, without limitation, an enzyme that catalyzes an essential function in a host cell. Among such essential functions are, without limitation, DNA replication, transcription, translation and certain metabolic pathways. The target molecule may also be a protein that carries out an essential cellular function in a host cell. For example, the target molecule may be a protein that is required for the structural integrity of the host cell. Alternatively, the target molecule may be a nucleic acid encoding an essential protein or an essential enzyme.

A host cell may comprise multiple target molecules. In theory, every essential component of a host cell may be a target molecule of a peptide. That is, the method of the invention may be used to identify multiple target-binding peptides in a single experiment, wherein each peptide binds to a different target molecule.

In certain embodiments, a peptide may be identified as a target-binding peptide without knowing the exact target molecule. That is, a peptide may be defined to specifically bind to an endogenous target molecule of a host cell based on its ability to inhibit growth of said host cell, but without knowing the exact target molecule of the peptide.

In certain embodiments, the target molecule may be an essential component of a pathogen. The term "pathogen", as used herein, is defined as any biological agent that causes disease or illness to its host. In certain embodiments, the pathogen may be a microbial pathogen or a parasite. In such embodiments, it is envisioned that binding of a peptide to an essential target molecule of the microbial pathogen or parasite inhibits the target molecule and thereby causes growth arrest and/or cell death of the pathogen or parasite. For example, specific binding of a peptide to a nucleic acid encoding an essential protein or enzyme may inhibit synthesis of said essential protein or enzyme and thereby inhibit growth of the pathogen. Alternatively, specific binding of a peptide to an essential protein or enzyme may inhibit the function of said essential protein or enzyme and thereby inhibit growth of the pathogen. Consequently, specific binding of a peptide to an endogenous target molecule may be identified by monitoring the growth behavior of host cells expressing a particular peptide candidate. Thus, in certain embodiments, the method according to the invention may be used for the identification of novel antimicrobial peptides, including antibiotic, antifungal and antiparasitic peptides. Specific pathogens that may be used as host cells in the method of the present invention are discussed herein.

### 1.2 Heterologous target molecules

In other embodiments, the target molecule may be a component of a heterologous cell. The term "heterologous", as used herein, refers to component that is derived from a source other than the endogenous source (the host cell), preferably from a different organism. In certain embodiments, the heterologous target molecule may be derived from a mammal. In certain embodiments the heterologous target molecule may be a mammalian protein or a mammalian nucleic acid. In certain embodiments, the heterologous target protein may be a human protein or a human nucleic acid. In other embodiments, the target molecule may be derived from a pathogen. The term "pathogen" as used herein comprises, without limitation, bacteria, viruses, fungi, prions, protozoa and uni- and multicellular parasites. In certain embodiments, the heterologous target molecule is a protein or a nucleic acid derived from a pathogen.

In such embodiments, specific binding of a peptide to the heterologous target molecule as such is unlikely to affect the growth behaviour or any other functionality of the host cell. Thus, enabling the identification of peptides that specifically bind to a heterologous target molecule may require the use of a biosensor that is functionally linked and/or comprises the heterologous target molecule or a fraction thereof. For example, the heterologous target molecule may be fused to a protein which activates expression of a reporter gene upon specific binding of a peptide to the heterologous target molecule. Examples of biosensors that may be fused or functionally linked to a target molecule are given below:

### 1.2.1 Two-hybrid system-based biosensors

The term "two-hybrid system" refers to a system comprising two chimeric molecules, one of which bears a nucleic acid binding region, the other of which bears an expression control element (e.g. a transactivation or repressor domain). The molecules further comprise a cognate binding pair such that one chimeric molecule is capable of specifically binding to the other chimeric molecule.

In certain embodiments, the method according to the invention may be used for the identification of peptides that inhibit the binding of a target molecule to its cognate binding partner. Such peptides may, for example disrupt protein-protein interactions that are involved in the manifestation and/or progression of various diseases, e.g. through the deregulation of signalling cascades and/or abnormal protein-protein interactions. In such embodiments, the biosensor may be a two-hybrid system, wherein the target molecule is fused to a first fragment of a transcription factor and wherein the cognate binding partner of the target molecule is fused to a second fragment of a transcription factor. Binding of the target molecule to its cognate binding partner will result in complementation of the transcription factor, which, in turn, may result in the expression of a reporter gene. Alternatively, complementation of the transcription factor may result in the expression of a repressor molecule that inhibits expression of a reporter gene. Thus, the skilled person is aware that two-hybrid-based reporter systems may be designed to either activate or inactivate expression of a reporter gene upon complementation of the components of the two-hybrid system.

Expression of a peptide that specifically binds to the target molecule may inhibit interaction with the cognate binding partner of the target molecule and thereby prevent complementation of the transcription factor. Consequently, binding of a peptide to a target molecule may alter the expression of a reporter gene, as described above, and may thereby result in a change in the abundance of a measurable output signal.

Two-hybrid systems may be used for the identification of peptides that inhibit disease-relevant protein-protein interactions in mammals and, in particular, in humans. Several diseases have been described in the art to be caused, at least in part, by abnormal protein-protein interactions or aggregations (Enno Klussmann and John Scott, Protein-Protein Interactions as New Drug Targets, Springer Berlin Heidelberg (2008); Scott et al., Small molecules, big targets: drug discovery faces the protein-protein interaction challenge, Nature Reviews Drug Discovery volume 15, pages 533-550 (2016); Rezwan and Auerbach, Yeast "N"-hybrid systems for protein-protein and drug-protein interaction discovery, Methods (San Diego, Calif.), 2012, 57(4):423-429). Accordingly, the method of the invention may be used to identify peptides that can be used for the treatment of such diseases.

Two-hybrid system-based biosensors that may be used in the method according to the present invention have been described in the art. Such a biosensor typically uses a yeast two hybrid system that reflect a key protein-protein interaction of a signaling pathway that is deregulated in a disease (e.g. cancer cell, for examples see Hamdi and Colas, Yeast two-hybrid methods and their applications in drug discovery, Trends Pharmacol Sci. 2012 Feb;33(2):109-18). Such a biosensor can then be used to identify molecules disrupting this specific protein-protein interaction and thus to identify molecules that are candidates for the treatment of the disease.

Besides two-hybrid systems as discussed above, the biosensor may also be a one-hybrid system or a three-hybrid system. For example, one-hybrid systems, such as bacterial one-hybrid systems and yeast one-hybrid systems, may be used preferably to identify peptides that interrupt protein-DNA interactions. Three hybrid-systems may be used preferably to identify peptides that interrupt protein-RNA interactions.

### 1.2.2 Enzyme-based biosensors

In other embodiments, a biosensor may only be functionally, but not covalently, linked to a target molecule. For example, the method according to the invention may be used to identify peptides that inhibit disease-relevant enzymes, such as novel or optimized protease inhibitors. Peptides that inhibit a particular protease may be identified in a host cell that expresses a) the protease and b) a biosensor protein comprising a cleavage site that is specifically recognized by said protease. As described above, the sensor may be, without limitation, a transcription factor. Thus, in the absence of an inhibitory peptide, said transcription factor may be degraded by the protease, thereby diminishing expression of a reporter gene. In the presence of an inhibitory peptide, on the other hand, the transcription factor is intact and induces expression of the reporter gene. Accordingly, peptides that inhibit the activity of the protease can be identified based on the higher expression level of the reporter gene in the host cell, which is reflected in a change of abundance of a measurable output signal.

It is to be understood that the reporter system may also be arranged in a way that expression of an inhibitory peptide results in decreased expression of the reporter gene. For example, the sensor molecule may be a repressor molecule that only inhibits expression of a reporter gene when the protease is inactivated through specific binding of a peptide.

Several proteases have been described in the art to be involved in the manifestation or progression of human diseases. Examples include, without limitation, cancer, Alzheimer's disease, arthritis, blood clotting disorders, allergies and infection. Accordingly, the method according to the invention may be used to identify peptides that can be used for the treatment of such diseases.

In certain embodiments, a method for identifying a candidate peptide that inhibits a disease-relevant peptidase may comprise the following steps:

Generation of the biosensor:

1) Selection of a disease-relevant protease.
2) Expression of the protease within a microbial host (e.g. a yeast cell).
3) Coupling of the protease substrate to a reporter. In certain embodiments, a protease cleavage site may be integrated such that the reporter (e.g. a transcription factor or an antibiotic resistance protein) is inactivated upon cleavage by the protease. In other embodiments, the reporter (e.g. a transcription factor or an antibiotic resistance protein) may be attached to a degradation tag and the protease cleavage site may be located in the interconnecting region between the degradation tag and the reporter. Thus, in cases where the protease cleavage site cleaved, the degradation tag is removed and the reporter is stable, otherwise it is degraded quickly by the native protein recycling pathways within the cell.
4) Expression of the reporter construct within the same host expressing the protease. This can be on the same plasmid or on a different plasmid.

Screening with the biosensor:

1) Expression of library of candidate peptides or a library of candidate complexes of non-ribosomal peptide biosynthesis enzymes within the biosensor, wherein preferably each cell produces a different peptide.
2) Cultivation of all cells in batch under selective conditions such that cells where the peptide inhibits the activity of the protease cannot grow anymore.
3) Sampling of the culture at different timepoints at the beginning of the culture and at least once after induction of peptide expression.
4) Next generation sequencing of all peptide-encoding DNA sequences to identify sequences that were depleted from the pool.

**2. The measurable output signal**

Within the present invention, specific binding of a peptide to a target molecule results in a change in the abundance of measurable output signals that can be assigned to said particular peptide or target molecule. A measurable output signal may be any signal that can be quantified in a sample, such as the number of host cells in a sample. It is preferred herein that measurable output signals are signals that can be assigned to a particular peptide or library member.

In certain embodiments, the measurable output signal is quantified by next-generation sequencing. That is, a measurable output signal is preferably a sequencing read that can be assigned to a particular library member. The abundance of sequencing reads assigned to a particular library member is determined at at least two time points where the first time point is before or shortly after the induction step.

Accordingly, determining the abundance of sequencing reads in a sample for a particular library member may comprise the following steps:

i) collecting cell samples from a culture at at least two time points; and
ii) isolating the library member-encoding DNA from the sample; and
iii) sequencing the isolated library-member encoding DNA samples by next-generation sequencing; and
iv) assigning the reads obtained by next-generation sequencing to library members; and
v) counting the sequencing reads at each time point assigned to each library member.

To determine whether a peptide is a target-binding peptide, the abundance of sequencing reads for that peptide has to be compared between at least two time points. To determine changes in the abundance of the measurable output signal, it is preferred that all samples are adjusted to a similar number of host cells, or are adjusted to a similar number of sequencing reads or are normalized using other statistical methods known in the art (e.g. using the negative binomial distribution). In certain embodiments, changes in the abundance of measurable output signals are determined using negative binomial distribution. Statistical tools for determining changes in the abundance of measurable output signals using negative binomial distribution are known in the art and include, without limitation, the DeSeq2 tool.

Within the present invention, it is preferred that the change in abundance for a particular library member at different time points of sampling either corresponds to cell viability or to the expression of a reporter gene, in particular a DNA modification enzyme.

When changes in the abundance of measurable output signals for a particular library member correspond to cell viability, every sequencing read that comprises a sequence encoding a peptide candidate or a candidate complex of non-ribosomal peptide biosynthesis enzymes, or fragments thereof, is considered a measurable output signal.

In embodiments where changes in the abundance of measurable output signals correspond to the expression of a DNA modifying enzyme, a measurable output signal is preferably a sequencing read that comprises (a) a sequence encoding a peptide candidate or a candidate complex of non-ribosomal peptide biosynthesis enzymes, or fragments thereof, and in addition (b) a DNA target sequence. That is, a sequencing read preferably comprises sequence information about a particular peptide candidate or candidate complex of non-ribosomal peptide biosynthesis enzymes and in addition on the degree of modification of the DNA target sequence.

It is to be understood that next-generation sequencing will also result in reads that cannot be assigned to a particular library member because they only comprise generic sequence information that is shared by all library members. Such reads may or may not be considered in the determination of the abundance of measurable output signals.

## 2.1 Correlation between cell viability and the abundance of measurable output signals

As described above, the abundance of measurable output signals for a particular library member may correlate with the abundance of host cells comprising said particular library member in a sample.

In certain embodiments, binding of a peptide to its target molecule directly affects the viability of the host cell. In such embodiments, the target molecule may be an essential molecule of the host cell which cannot carry out its normal function when specifically bound by a peptide, thereby resulting in growth retardation, arrest and/or cell death. In such embodiments, binding of a peptide to its target molecule may result either in the lysis of host cells encoding said peptide or in a growth arrest. Since host cells expressing non-active or weakly-active peptides will not or to a lesser extend be affected in their growth behaviour, host cells expressing peptides that inhibit essential cell component will consequently be diluted out of a culture, resulting in a lower abundance of measurable output signals for library members encoding target-binding peptides.

It has to be noted that the invention also encompasses embodiments where the binding of a peptide to a target molecule is indirectly linked to cell viability. As described in more detail above, non-essential target molecules, such as heterologous target molecules, may be comprised in or functionally linked to a biosensor. Upon binding of a peptide to a target molecule comprised in or functionally linked to a biosensor, the biosensor may alter the expression of a reporter gene. The skilled person is aware of methods to design biosensor systems that either induce or suppress the expression of a reporter gene upon specific binding of a peptide.

In certain embodiments, the reporter gene may be a gene that influences viability of the host cell. For example, the reporter gene may encode a toxin that kills the host cell when its expression is activated by the biosensor. Alternatively, the reporter gene may encode a selection marker, such as an antibiotic resistance gene or an auxotrophic marker, which allows the host cell to grow under selective conditions.

In certain embodiments, the reporter gene may encode a toxic protein. In such embodiments, binding of a peptide to a target molecule comprised in or functionally linked to a biosensor may result in expression of the toxin and, thus, in cell death. Accordingly, the abundance of measurable output signals for a target-binding peptide would be expected to be lower at the second time point, i.e. after inducing the expression of the peptide.

In other embodiments, the reporter gene may be an antibiotic resistance gene or an auxotrophy marker. In such embodiments, the reportergene may be expressed in the absence of a target-binding peptide, while specific binding of a peptide to the target molecule may suppresses the expression of the reporter gene. Consequently, specific binding of a peptide to a target molecule comprised in or functionally linked to a biosensor may result in growth arrest and/or cell death when selective pressure is applied. In such embodiments, the abundance of measurable output signals for a target binding peptide would be expected to be lower after inducing the expression of the peptide.

However, it is known in the art that reporter systems may also be designed such that the expression of a reportergene is induced when a peptide specifically binds to a target molecule. Accordingly, a reporter system may be designed such that growth of a host cell is only supported upon binding of a peptide to a target molecule. In such embodiments, host cells expressing target-binding peptides would be expected to accumulate in a culture and the abundance of measurable output signals for a target-binding peptide would consequently be higher after inducing the expression of the peptide.

Accordingly, in a particular embodiment, the invention relates to a method for identifying a peptide which binds specifically to a target molecule, the method comprising the steps of:

(a) introducing a DNA library into a plurality of host cells, the DNA library comprising at least two members, wherein each of the at least two DNA library members comprises a nucleic acid sequence encoding

(i) a peptide candidate; or

(ii) a candidate complex of non-ribosomal peptide biosynthesis enzymes; wherein the nucleic acid sequence encoding the peptide candidate or the candidate complex of non-ribosomal peptide biosynthesis enzymes is operably linked to an inducible promoter;

wherein the host cell comprises the target molecule, and wherein specific binding of a peptide to said target molecule results in a change in the abundance of the library member encoding said peptide;

(b) culturing the plurality of host cells obtained in step (a);

(c) inducing the synthesis of the peptide candidates and/or the candidate complex of non-ribosomal peptide biosynthesis enzymes in the plurality of host cells cultured in step (b);

(d) collecting a first sample at a first time point from the culture of step (b) before the induction step (c) and collecting a second sample at a second time point from the culture of step (b) after the induction step (c);

(e) determining the abundance of a library member encoding a peptide in the samples taken at the first time point and at the second time point; and

(f) identifying

(i) a peptide candidate to be a target binding peptide if the abundance of the library member encoding said peptide candidate is different between the first time point and the second time point; and/or

(ii) a candidate complex of non-ribosomal peptide biosynthesis enzymes to synthesize a target binding peptide if the abundance of the library member encoding said candidate complex of non-ribosomal peptide biosynthesis enzymes is different between the first time point and the second time point.

As used herein, the term "antibiotic resistance gene" refers to a gene conferring resistance to antibiotics, and the cells comprising this gene survive even in the environment treated with the corresponding antibiotic. Therefore, the antibiotic resistance gene is effectively used as a selection marker for cells that express a target-binding peptide. Specifically, the resistance genes against ampicillin, tetracycline, kanamycin, chloramphenicol, streptomycin, or neomycin may be used. It has to be noted that the term "antibiotic resistance gene" is used in its broadest sense and does not exclusively refer to genes that confer resistance against antibiotics to bacterial cells. Instead, the term antibiotic resistance gene also encompasses selection markers that confer resistance against toxic molecules to eukaryotic cells. Toxins that may be used for the selection of eukaryotic cells include, without limitation, geneticin (G418 sulfate), zeocin, hygromycin B, puromycin, and blasticidin. Thus, the term "antibiotic resistance gene" may be used interchangeably with the term "selection marker".

The term "auxotrophic marker" refers to a nucleotide sequence which, when expressed by the host cell, allows the host cell to manufacture a particular nutrient (usually a metabolite, vitamin or an amino acid) endogenously. The marker serves as a positive selective aid when sequences carrying it are introduced into a host cell that is unable to manufacture the relevant nutrient.

**2.2 Correlation between DNA modifications and the abundance of measurable output signals**

In certain embodiments, the reporter gene may encode a DNA modifying enzyme. That is, binding of a peptide to a target molecule comprised in or functionally linked to a biosensor may result in the expression of a DNA modifying enzyme (or inhibit the expression of a DNA modifying enzyme). In such embodiments, it is preferred that the host cells comprise a DNA target sequence which can be modified by the DNA modifying enzyme. More preferably, the DNA target sequence is comprised in the DNA library, such that information on the degree of modification and the identity of the peptide candidate or the candidate complex of non-ribosomal biosynthesis enzymes can be retrieved from a single sequencing read. As described above, the abundance of measurable output signals for the library members may be determined based on all reads that allow identification of the peptide/complex of non-ribosomal biosynthesis enzymes and the degree of modification of the DNA target sequence.

Accordingly, in a particular embodiment, the invention relates to a method for identifying a peptide which binds specifically to a target molecule, the method comprising the steps of:

(a) introducing a DNA library into a plurality of host cells, the DNA library comprising at least two members, wherein each of the at least two DNA library members comprises a nucleic acid sequence encoding

(i) a target sequence;

(ii) a peptide candidate or a candidate complex of non-ribosomal peptide biosynthesis enzymes; wherein the nucleic acid sequence encoding the peptide candidate or the candidate complex of non-ribosomal peptide biosynthesis enzymes is operably linked to an inducible promoter;

wherein the host cell comprises the target molecule and a DNA modifying enzyme, and wherein specific binding of a peptide to said target molecule results in a modification of the target sequence by the DNA modifying enzyme;

(b) culturing the plurality of host cells obtained in step (a);

(c) inducing the synthesis of the peptide candidates and/or the candidate complex of non-ribosomal peptide biosynthesis enzymes in the plurality of host cells cultured in step (b);

(d) collecting a first sample at a first time point from the culture of step (b) before the induction step (c) and collecting a second sample at a second time point from the culture of step (b) after the induction step (c);

(e) determining the degree of modification of the target sequence for at least one library member in samples taken at the first time point and at the second time point; and

(f) identifying

(i) a peptide candidate to be a target binding peptide if the degree of modification of the target sequence in the library member encoding said peptide candidate is different between the first time point and the second time point; and/or

(ii) a candidate complex of non-ribosomal peptide biosynthesis enzymes to synthesize a target binding peptide if the degree of modification of the target sequence in the library member encoding said candidate complex of non-ribosomal peptide biosynthesis enzymes is different between the first time point and the second time point.

DNA modifying enzymes that may be used in the method of the invention include, without limitation, DNA recombinases, DNA methyltransferases or DNA nucleases. Preferably, the DNA modification is a modification that can be detected by a sequencing method known in the art.

The term "recombinase," as used herein, refers to a site-specific enzyme that mediates the recombination of DNA between recombinase recognition sequences, which results in the excision, integration, inversion, or exchange (e.g., translocation) of DNA fragments between the recombinase recognition sequences. Recombinases can be classified into two distinct families: serine recombinases (e.g., resolvases and invertases) and tyrosine recombinases (e.g., integrases). Examples of serine recombinases include, without limitation, Hin, Gin, Tn3, $\beta$-six, CinH, ParA, $\gamma\delta$, Bxb1, $\phi$C31, TP901, TG1, $\phi$BT1, R4, $\Phi$RV1, $\phi$FC1, MR11, A118, U153, and gp29. Examples of tyrosine recombinases include, without limitation, Cre, FLP, R, Lambda, HK101, HK022, and pSAM2. The skilled person is aware of target sequences that can be recognized by a specific recombinase. The degree of modification of the target sequence by a recombinase may indicate the level of biosensor activity and, consequently, the binding specificity of the peptide encoded by a host cell.

As used herein, the term "DNA methyltransferase" family of enzymes catalyze the transfer of a methyl group to DNA. Three active DNA methyltransferases have been identified in mammals, including DNMT1, DNMT3A and DNMT3B. The skilled person is aware of target sequences that may be modified by a DNA methyltransferase. The activity of a biosensor, and thus the binding specificity of a peptide candidate, may be determined based on the degree of methylation of the target sequence. Sequencing methods that are suitable for determining the degree of methylation of a target sequence are known in the art.

### 2.3 Identification of target-binding peptides

As disclosed in more detail above, a library member is identified to encode a target-binding peptide if the abundance of measurable output signals for said library member is either lower or higher after the induction step. The decision whether the abundance of measurable output signals has to be higher or lower after the induction step in order to identify a library member as encoding a target-binding peptide depends mainly on the choice of the reporter system.

In embodiments where the specific binding of a peptide results in growth arrest and/or cell death of the host cell, the abundance of measurable output signals for library members that encode a target-binding peptide is expected to be lower after the induction step. In such embodiments, a library member is identified to encode a target-binding peptide, if the abundance of measurable output signals for said library after the induction step is at least 10%, at least, 20%, at least 30%, at least 40%, at least 50%, at least 60%, at least 70%, at least 80%, at least 90% or 100% lower than before the induction step. That is, in certain embodiments, where a peptide efficiently kills its host cell, no measurable output signal will be detected after the induction step for the library member encoding said peptide.

In other embodiments, the host cell can only grow under selective conditions upon specific binding of a peptide to a target molecule. In such embodiments, a library member is identified to encode a target-binding peptide, if the abundance of measurable output signals for said library member after the induction step is at least 10%, at least, 20%, at least 30%, at least 40%, at least 50%, at least 60%, at least 70%, at least 80%, at least 90% or at least 100% higher than before the induction step.

In certain embodiments, specific binding of a peptide to a target molecule results in the expression of a DNA modifying enzyme. Certain DNA modifying enzymes generate a binary signal. For example, a DNA target sequence may comprise a recombinase recognition site such that the DNA target sequence will be flipped upon expression of the DNA modifying enzyme. Alternatively, the DNA target sequence may comprise a single methylation site such that the DNA target sequence will methylated upon expression of the DNA modifying enzyme. In such cases, the measurable output signal may be a sequencing read that can be assigned to a peptide/complex of non-ribosomal peptide biosynthesis enzymes and at the same time comprises a modified DNA target sequence.

In certain embodiments, specific binding of a peptide to a target molecule induces the expression of a DNA modifying enzyme. In such embodiments, a peptide may be identified to be a target-binding peptide if, after the induction step, the abundance of measurable output signals that can be assigned to a peptide/complex of non-ribosomal peptide biosynthesis enzymes and at the same time comprise a modified DNA target sequence is at least 10%, at least, 20%, at least 30%, at least 40%, at least 50%, at least 60%, at least 70%, at least 80%, at least 90% or at least 100% higher than before the induction step.

In other embodiments, specific binding of a peptide to a target molecule may inhibit the expression of a DNA modifying enzyme. In such embodiments, a peptide may be identified to be a target-binding peptide if, after the induction step, the abundance of measurable output signals that can be assigned to a peptide/complex of non-ribosomal peptide biosynthesis enzymes and at the same time comprise a modified DNA target sequence is at least 10%, at least, 20%, at least 30%, at least 40%, at least 50%, at least 60%, at least 70%, at least 80%, at least 90% or 100% lower than before the induction step.

In certain embodiments, the DNA target sequence comprises more than one modification site. That is, the DNA target sequence may, for example, comprise two or more methylation sites. In such embodiments, the measurable output signal may be defined to be a sequencing read that can be assigned to a peptide/complex of non-ribosomal peptide biosynthesis enzymes and at the same time comprises at least a defined number of DNA modifications.

In certain embodiments, more than one sample is collected and analyzed after the induction step. In such embodiments, a library member may be identified to encode a target-binding peptide if the abundance of measurable output signals for said library member determined at least one time point after the induction step is different compared to the first time point. In certain embodiments, a library member may be identified to encode a target-binding peptide if the abundance of measurable output signals for said library member determined at least one time point after the induction step is higher compared to the first time point. In certain embodiments, a library member may be identified to encode a target-binding peptide if the abundance of measurable output signals for said library member determined at each time point after the induction step is higher compared to the first time point. In certain embodiments, a library member may be identified to encode a target-binding peptide if the abundance of measurable output signals for said library member determined at least one time point after the induction step is lower compared to the first time point. In certain embodiments, a library member may be identified to encode a target-binding peptide if the abundance of measurable output signals for said library member determined at each time point after the induction step is lower compared to the first time point.

### 3. The host cell

The method of the present invention is carried out in a host cell. The term "host cell," as used herein means any cell, prokaryotic or eukaryotic, including animal and plant cells, that may be transformed or transfected with the DNA library of the invention.

In certain embodiments, the host cell is a bacterial host cell. The term "bacterial host cell" includes both Gram-negative and Gram-positive microorganisms. In certain embodiments, the bacterial host cell is *Escherichia coli* (*E. coli*). In certain embodiments, *E. coli* may be used in the screening of peptides that bind to heterologous target molecules, for example heterologous target molecules that are comprised in or functionally linked to a biosensor, as described herein. In other embodiments, *E. coli* may be used in the screening of peptides that bind to endogenous target molecules. Several *E. coli* strains, such as, without limitation, enteropathogenic *E. coli* (EPEC), enterohaemorrhagic *E. coli* (EHEC), enterotoxigenic *E. coli* (ETEC), enteroaggregative *E. coli* (EAEC), enteroinvasive *E. coli* (EIEC) and diffusely adherent *E. coli* (DAEC), have been described to be pathogenic in humans. Accordingly, the method according to the invention may be used for the identification of peptides that inhibit essential target molecules of *E. coli* and may thus be used in the therapy of *E. coli* infections. In certain embodiments, the essential endogenous target molecules of *E. coli* may be a conserved in other bacteria. In such embodiments, inhibitory peptides that have been identified in *E. coli* may not only be useful in the treatment of *E. coli* infections, but may also be useful in the treatment of infections with closely or even more distantly related bacterial strains.

Besides *E. coli,* the method according to the invention may be performed with other bacterial model organisms, such as, without limitation *Pseudomonas aeruginosa, Klebsiella pneumonia, Acinetobacter* spp., *Escherichia coli, Enterobacter* spp., *Staphylococcus aureus* and *Enterococcus* spp.

In certain embodiments, the method of the invention may be carried out directly in a pathogenic bacterium. The term "pathogenic bacteria" as used herein, means any bacteria which are involved in the pathogenesis of a disease. Preferably, the pathogenic bacterium is a bacterium that can be efficiently transformed with a DNA library. Bacterial strains that may be used in the method according to the invention include *Pseudomonas aeruginosa, Klebsiella pneu-*

*monia, Acinetobacter* spp., *Escherichia coli, Enterobacter* spp., *Staphylococcus aureus* and *Enterococcus* spp.

When the method according to the invention is performed in pathogenic bacteria, it is preferred that the method is used for the identification of peptides that bind to endogenous target molecules. More preferably, the method according to the invention may be used for the identification of peptides that inhibit endogenous target molecules of pathogenic bacteria. Thus, the method according to the invention may be used to identify peptides that can be used in the treatment of bacterial infections caused by the host cell.

In further embodiments, the host cell may be a eukaryotic host cell. That is, in certain embodiments, the host cell may be a yeast. The term "yeast" as used herein preferably refers to yeasts of the genus *Saccharomyces,* particularly preferably to the species *Saccharomyces cerevisiae* or *Saccharomyces bayanus*. This also includes subspecies such as *Saccharomyces cerevisiae* subsp. *bayanus.* However, other yeasts may be used in the claimed method.

Yeasts may be used for the identification of peptides that bind to an endogenous target molecule of said yeast. Such peptides may be used, for example, in the treatment of yeast/fungal infections. Alternatively, yeasts may be used as platform for identifying peptides that bind to heterologous target molecules that are comprised in or functionally linked to a biosensor, as described herein. In certain embodiments, yeasts may be used as host cells to identify peptides that bind to human target molecules, such as human target molecules that are involved in the manifestation or progression of a human disease. Such humanized yeast biosensors have been disclosed in the art, for example by Laurent et al. (Efforts to make and apply humanized yeast; Briefings in Functional Genomics, Volume 15, Issue 2, March 2016, Pages 155-163) and Martin-Yken (Yeast-Based Biosensors: Current Applications and New Developments; Biosensors 2020, 10(5), 51; https://doi.org/10.3390/bios10050051), and may be used within the method according to the invention.

In other embodiments, further unicellular pathogens, such as unicellular fungi or parasites, may be used as host cells in the method of the invention to identify peptides that inhibit essential functions of these pathogenic host cells. However, it has to be understood that the host cell has to be a host cell that can be efficiently transformed or transfected with a DNA library.

In further embodiments, the method according to the invention may be carried out in a mammalian host cell. The mammalian host cell may be any mammalian cell that can be efficiently transfected or transduced with a DNA library. Preferably, DNA libraries for use in mammalian cells exclusively encode ribosomal peptides.

Examples of mammalian host cells include, without limitation, HEK 293, CHO or HeLa. In certain embodiments, the mammalian host cell may be an engineered mammalian host cell. In certain embodiments, the engineered mammalian host cell may be a model for a human disease. A disease model is an animal or a cell displaying all or some of the pathological processes that are observed in the actual human or animal disease. Studying disease models aids understanding of how the disease develops and testing potential treatment approaches. Preferably, the disease model may be used to identify peptides that can be used in the treatment of the particular disease. Disease models that may be used in the method of the invention have been disclosed by Holliday and Speirs (Choosing the right cell line for breast cancer research; Breast Cancer Research volume 13, Article number: 215 (2011) and Slanzi et al. (In vitro Models of Neurodegenerative Diseases; Front. Cell Dev. Biol., 13 May 2020).

### 3.1 Culturing of the host cell

Within the present invention, it is envisioned that a plurality of host cells that have been transformed or transduced with a DNA library are cultured in an appropriate cell culture medium. The term "culturing" as used herein refers to incubating a host cell under conditions wherein the host cell can carry out biological processes. At least in certain embodiments of the present invention, peptide candidates are identified to specifically bind to a target molecule if they exert a growth inhibitory effect on their host cell, which consequently results in the dilution of such host cells from the cell culture. Accordingly, the term "culturing" as used herein preferably refers to incubating a host cell under conditions wherein the host cell can carry out biological processes and is able to proliferate. In certain embodiments, the cell culture medium is a liquid cell culture medium. In certain embodiments, the cell culture medium is a solid cell culture medium.

The skilled person is aware that the choice of cell culture medium depends on the choice of the host cell. However, the skilled person is aware of suitable cell culture media that may be used for culturing a particular host cell. Further, the skilled person is aware of suitable conditions for culturing a particular host cell. Cell culture conditions that may influence the proliferation behavior of a host cell include temperature, agitation and aeration.

### 4. The DNA library

Within the present invention, a DNA library is introduced into a host cell. The term "DNA library" is defined herein as a collection of polynucleotide molecules. In certain embodiments, the DNA library comprises a plurality of circular polynucleotides. The term "circular polynucleotide" as used herein refers to a nucleic acid in which no double-stranded DNA ends are present. In certain embodiments, the circular polynucleotide is a plasmid, a cosmid, a phagemid or an artificial chromosome, such as a bacterial artificial chromosome (BAC), a yeast artificial chromosome (YAC) or a P1 artificial chromosome (PAC). Preferably, the DNA library comprises a plurality of plasmids. The term "plasmid" as used herein refers to a circular double-stranded (ds) DNA construct used as a cloning vector, and which forms an extrachromosomal self-replicating genetic element in some eukaryotes or prokaryotes, or integrates into the host chromosome.

Circular polynucleotides preferably comprise an origin of replication that allows the circular polynucleotide to autonomously replicate in the host cell. Further, the circular polynucleotide may comprise a selection marker to separate host cells that received a library member from host cells that did not receive a library member. The skilled person is aware of origins of replication and of selection markers that are compatible with a particular host cell.

The DNA library may, in addition or alternatively, comprise a collection of linear polynucleotides. It is to be understood that linear polynucleotides may not be efficiently introduced and/or maintained in a host cell, as some host cells efficiently degrade foreign linear DNA molecules. Thus, DNA libraries comprising linear polynucleotides are preferably used in combination with host cells that can be efficiently transformed or transfected with linear polynucleotides and that can efficiently express polypeptides encoded on the linear polynucleotides. As in the case of the circular polynucleotide, a linear polynucleotide may comprise a selection marker to allow selection of host cells that received a linear polynucleotide. Additionally, linear polynucleotides, may comprise one or more homology region(s) which facilitate the integration of the linear polynucleotides into the host cell genome.

The skilled person is aware of methods to design DNA libraries that can be efficiently expressed in a particular host cell. In certain embodiments, the host cell may be a bacterial host cell and the DNA library may comprise a collection of plasmids. In other embodiments, the host cell may be *E. coli* and the DNA library may comprise a collection of plasmids. In other embodiments, the host cell may be a pathogenic bacterium, such as any one of the pathogenic bacteria disclosed herein, and the DNA library may comprise a collection of plasmids. In other embodiments, the host cell may be a yeast and the DNA library may comprise a collection of plasmids. In other embodiments, the host cell may be *Saccharomyces cerevisiae* and the DNA library may comprise a collection of plasmids.

Within the present invention, the DNA library is used for the identification of target binding peptides. That is, the library may either encode two or more peptide candidates or, alternatively, may encode two or more complexes of enzymes that catalyze the synthesis of non-ribosomal peptides. Accordingly, the method of the invention may be used for the discovery of target-binding ribosomal or non-ribosomal peptides. Within the present invention, a library member is said to encode a ribosomal peptide, if the peptide is encoded by a nucleic acid that is transcribed and translated into a protein by the host cell. However, it has to be noted that ribosomal peptides may be modified post-translationally by host cell enzymes or heterologous enzymes. A library member is said to encode a non-ribosomal peptide, if the peptide is synthesized by a complex of non-ribosomal peptide biosynthesis enzymes that is encoded in the library member.

The DNA library used in the method according to the present invention may encode a plurality of ribosomal and/or non-ribosomal peptides. That is, the DNA library may encode at least two different ribosomal and/or non-ribosomal peptides. In certain embodiments, the DNA library encodes at least 100 different ribosomal and/or non-ribosomal peptides. In certain embodiments, the DNA library encodes at least 1'000 different ribosomal and/or non-ribosomal peptides. In certain embodiments, the DNA library encodes at least 2'500 different ribosomal and/or non-ribosomal peptides. In certain embodiments, the DNA library encodes at least 5'000 different ribosomal and/or non-ribosomal peptides. In certain embodiments, the DNA library encodes at least 7'500 different ribosomal and/or non-ribosomal peptides. In certain embodiments, the DNA library encodes at least 10'000 different ribosomal and/or non-ribosomal peptides. Two ribosomal and/or non-ribosomal peptides are said to be different if they differ in their amino acid sequences.

It is preferred within the present invention, that a library member does not encode more than one peptide candidate or more than one candidate complex of non-ribosomal peptide biosynthesis enzymes. More preferably, each library member encodes exactly one peptide candidate or exactly one candidate complex of non-ribosomal peptide biosynthesis enzymes. However, it has to be noted that due to occasional errors during the preparation of a DNA library, library members encoding more than one peptide candidate or more than one candidate complex of non-ribosomal peptide biosynthesis enzymes may be obtained. In addition, library members encoding no peptide candidate or only a partial complex of non-ribosomal peptide biosynthesis enzymes may be obtained. Thus, it is preferred herein that at least 50%, at least 60%, at least 70%, at least 80%, at least 90% or at least 95% of the library members encode one ribosomal peptide or one complex of non-ribosomal peptide biosynthesis enzymes.

**4.1 Synthesis of DNA libraries**

The skilled person is aware of methods to generate a DNA library for use in the method according to the present invention. For example, a plurality of nucleotide sequences encoding peptide candidates and/or complexes of non-ribosomal peptide biosynthesis may be retrieved from literature/databases and/or may be designed computationally. A plurality of polynucleotides, wherein each polynucleotide encodes a peptide candidate or a complex of non-ribosomal peptide biosynthesis enzymes, may then be synthesized chemically based on the retrieved and/or designed nucleotide sequences. Preferably, these polynucleotides comprise additional sequences, such as restriction sites, that allow for cloning of the nucleic acid molecules into a DNA vector. The skilled person is aware of commercial providers that offer custom synthesis of DNA molecules. The chemically synthesized polynucleotides may then be cloned into a DNA vector, preferably wherein the DNA vector comprises an inducible promoter that will be operably linked to the polynucleotide after the cloning step.

Alternatively, polynucleotides encoding candidate peptides may be amplified from a template by any method known in the art, such as polymerase chain reaction, and assembled into a DNA vector.

### 4.2 Introduction of the DNA library into host cells

Within the method according to the present invention, a DNA library is introduced into a host cell. The term "introduced" as used herein refers to providing a nucleic acid (e.g., a library member) to a cell. The term "introduced" also includes reference to the incorporation of a nucleic acid into a eukaryotic or prokaryotic cell where the nucleic acid may be incorporated into the genome of the cell, and includes reference to the transient provision of a nucleic acid to the cell. "Introduced" further includes reference to stable or transient transformation methods, as well as sexually crossing. Thus, "introduced" in the context of inserting a nucleic acid fragment (e.g., a recombinant DNA construct/expression construct) into a cell, means "transfection" or "transformation" or "transduction" and includes reference to the incorporation of a nucleic acid fragment into a eukaryotic or prokaryotic cell where the nucleic acid fragment may be incorporated into the genome of the cell (e.g., chromosome, plasmid, plastid, or mitochondrial DNA), converted into an autonomous replicon, or transiently expressed.

The skilled person is aware of various methods to introduce foreign DNA into a host cell. Further, the skilled person is aware which methods are suitable for introducing a DNA library into a particular host cell and which requirements a DNA library has to fulfil to be compatible with a particular host cell.

In certain embodiments, a plasmid library is introduced into a bacterial host cell, such as *E. coli,* by transformation. The term "transformation", as used herein, refers to the introduction of an exogenous DNA material into a host cell in which the exogenous DNA material is replicable as an element separated from or incorporated into the host genome. Resulting from the transformation of the vector into a host cell, the transformant anchors the vector in the form of a plasmid or as is incorporated into the chromosome of the host cell. While certain host cells are naturally competent to take up foreign DNA, other host cells have to be made chemically competent. Introduction of foreign DNA into host cells may also be achieved by physical means, for example via electroporation.

In other embodiments, yeast cells are transformed with a plasmid library. Protocols for transforming DNA libraries into yeast cells have been disclosed in the art.

In other embodiments, DNA libraries comprising circular or linear polynucleotides may be transfected into a mammalian host cell. The term "transfection" as used herein refers to the introduction of foreign DNA into eukaryotic cells. Transfection may be accomplished by a variety of means known in the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics.

In other embodiments, the DNA library may comprise viral vectors and the DNA library may be introduced into a prokaryotic or eukaryotic host cell via transduction. The term "transduction" as used herein means a process in which a foreign DNA is introduced into a cell using a vector such as viral vector, plasmid vector, or the like.

### 4.3 Libraries comprising ribosomal peptides

The DNA library used in the method according to the invention may encode one or more ribosomal peptides. A ribosomal peptide is a peptide that is directly encoded by a polynucleotide and is synthesized by a host cell via transcription and translation of said polynucleotide. That is, the amino acid sequence of a ribosomal peptide corresponds to the nucleic acid sequence of the polynucleotide encoding said ribosomal peptide. Within the present invention, the term "peptide candidate" refers to a peptide that is encoded in a DNA library. A peptide candidate may be identified to be a target binding peptide by using the method according to the invention.

Ribosomal peptides typically have a size ranging from 5 to 100 amino acids. That is, the peptide candidates encoded in the DNA library according to the invention may have a size ranging from 5 to 100 amino acids, preferably from 10 to 75 amino acids, more preferably from 20 to 50 amino acids. Ribosomal peptides may consist of proteinogenic amino acids. However, certain amino acids comprised in a ribosomal peptide may be post-translationally modified. Post-translational modifications that are commonly found in ribosomal peptides include, without limitation, disulfide bridges, amidations, backbone cyclizations, thioether bridges, dehydrations, sidechain cyclizations, lipidations, terminal glutamate acetylations, glycosylations, methylations, epimerisations and hydroxylations. Post-translational modifications may be introduced by enzymes. These enzymes may be naturally found in the host cell used in the method of the present invention. In other embodiments, enzymes for introducing post-translational modification into a peptide may be introduced into the host cell by means of genetic engineering.

The ribosomal peptides encoded in the DNA library according to the invention may be naturally occurring peptides or may be synthetic peptides. A naturally occurring peptide is a peptide that has been previously found in nature. It is important to note that naturally occurring peptides also include predicted peptides which have been identified by DNA sequencing and/or bioinformatic methods. Polynucleotides encoding a naturally occurring peptide may be obtained directly from their origin or, more conveniently, by chemical synthesis based on sequence information retrieved from literature or sequence databases. A synthetic peptide, on the other hand, is a peptide that has not been described in nature before and has been obtained artificially. However, a synthetic peptide may also be a modified variant of a naturally occurring peptide. Certain synthetic peptides may later on be identified to be naturally occurring peptides. Synthetic peptides may be designed randomly, or may be designed in a targeted manner, for example by replacing certain amino acid residues in a template sequence.

## 4.4 Non-ribosomal peptides

The term "non-ribosomal peptide" as used herein refers to a peptide chain produced by one or more non-ribosomal peptide biosynthesis enzymes. Thus, as opposed to "ribosomal peptides", non-ribosomal peptides are not produced by a cell's ribosomal translation machinery. Polypeptides produced by such non-ribosomal peptide synthesis enzymes may be linear, cyclic or branched peptides. Numerous examples of non-ribosomal peptides that are produced by one or more non-ribosomal peptide biosynthesis enzymes are known in the art. One non-limiting example of non-ribosomal peptides that may be produced in accordance with the present invention is surfactin. Those of ordinary skill in the art will be aware of other non-ribosomal peptide biosynthesis enzymes that may be encoded in the DNA library of the present invention. Examples include without limitation, ciclosporin, vancomycin, colistin, polymyxin, penicillin, pedopeptin, octapeptin, tridecaptin, odilorhabdin, teixobactin, gramicidin, actinomycin, daptomycin, epothilone and bleomycin.

A "complex of non-ribosomal peptide biosynthesis enzymes" is defined herein as a group of enzymes that can catalyze the formation of a non-ribosomal peptide. It has to be noted that the term "complex" is to be understood in its broadest sense and does not necessarily refer to physical complexes driven by non-covalent bonds. However, two or more enzymes forming a complex of non-ribosomal peptide biosynthesis enzymes may form non-covalent bonds. In certain embodiments a "complex of non-ribosomal peptide biosynthesis enzymes" may consist of a single enzyme.

Within the present invention, it is preferred that all non-ribosomal peptide biosynthesis enzymes that are required for the synthesis of a particular non-ribosomal peptide are encoded in a single library member. That is, the entire complex of non-ribosomal peptide biosynthesis enzymes that is required for the synthesis of a particular non-ribosomal peptide is encoded in a single polynucleotide.

## 4.5 DNA libraries encoding antimicrobial peptides

In certain embodiments, the method according to the invention may be used for the identification of antimicrobial peptides. The term "antimicrobial", as used herein, refers to the ability to slow, reduce, terminate or inhibit the growth of microorganisms. Microorganisms include, but are not limited to, fungi, parasites and bacteria.

A vast number of (potentially) antimicrobial peptides, including ribosomal and non-ribosomal peptides, have been disclosed in the art. However, it is not always known whether an antimicrobial peptide is active against a certain species. Thus, the method according to the invention may be used for the identification of peptides that inhibit growth or kill a particular microbial species. For example, the method according to the invention may be performed in the host organism *E. coli* to identify peptides that act specifically against *E. coli*. Instead of *E. coli,* other host organisms may be used to identify antimicrobial peptides that are active against these other host organisms.

Several databases for antimicrobial peptide are known in the art. For example, the Database of Antimicrobial Activity and Structure of Peptides (DBAASP; https://dbaasp.org/) comprises sequences of over 16'000 antimicrobial peptides. Peptides from this database may be included as peptide candidates in the DNA library according to the invention.

Alternatively, or in addition, the DNA library may comprise peptides candidates that have been disclosed in the prior art, but which have not been tested for their antimicrobial activity. For example, tools such as *tblastn* (https://blast.ncbi.nlm.nih.gov/Blast.cgi?PROGRAM=tblastn&PAGE_TYPE=BlastSearch&LINK _LOC=blasthome) may be used to identify peptides with sequence homologies to known antimicrobial peptides. Such homologs may be included in the DNA library and tested for antimicrobial activity using the method according to the invention.

Alternatively, the method according to the invention may be used to identify improved variants of known antimicrobial peptides. That is, the amino acid sequence of a known antimicrobial peptide may be used as template when designing a DNA library. Designing the DNA library may include, without limitation, site-directed saturation mutagenesis of one or more amino acid residues of the template.

Further, the DNA library may encode computationally designed peptide candidates. Methods to design peptide candidates that specifically bind to a target molecule, for example to the active site of an enzyme, have been disclosed in the art. With the method of the present invention, various candidate peptides may be tested simultaneously to identify peptides that specifically bind to the target molecule.

Complexes of non-ribosomal peptide biosynthesis enzymes that catalyze the synthesis of antimicrobial peptides have been disclosed in the art. The skilled person is aware of methods to retrieve the nucleic acid sequences of the enzymes comprised in such complexes and to assemble them in a single nucleic acid molecule that can be expressed in a host cell. Examples of non-ribosomal peptides that may be included in the method according to the invention are summarized in the Norine database (https://bioinfo.lifl.fr/norine/).

It is to be understood that the DNA libraries used for the identification of antimicrobial peptides with the method according to the invention may comprise library members encoding ribosomal peptides and library members encoding non-ribosomal peptides. That is, the method according to the invention may be used to identify ribosomal peptides and non-ribosomal peptides in a single experiment.

**4.6 DNA libraries encoding therapeutic peptides**

In certain embodiments, the method according to the invention may be used for the identification of therapeutic peptides. The term "therapeutic peptide", as used herein, refers to a peptide comprising two or more amino acids but not more than 100 amino acids, covalently linked together through one or more amide bonds, wherein upon administration of the peptide to a subject, the subject receives a therapeutic effect (e.g., administration of the therapeutic peptide treats a cell, or cures, alleviates, relieves or improves a disease or disorder, or a symptom of a disease or disorder). It is to be understood that the term "therapeutic peptide", in its broadest sense, encompasses antimicrobial peptides. However, the term "therapeutic peptide" is preferably used herein for peptides that target cells or other components of a subject in need. Within the present invention, a "therapeutic peptide" may also be referred to as a peptide drug.

As described above, the method according to the invention may be used for the identification of peptides that inhibit the formation of protein-protein interactions, protein-DNA interaction or protein RNA-interactions. Such peptides may be used in the treatment of diseases or disorders that result from the abnormal interaction and/or aggregation of proteins. Host cells that mimic such disease models are known in the art and may be used in the method according to the invention. For example, two-hybrid systems may be designed to mimic such disease models in a host cell and may be used as recombinant biosensors in the method according to the invention.

Alternatively, the method according to the invention may be used to identify peptides that inhibit the activity of an enzyme that is involved in the manifestation or progression of a disease or disorder in a subject. For example, peptides that inhibit the activity of a particular protease may be identified with the method according to the invention. This may be achieved, for example, in host cells that express the particular protease and, in addition, a biosensor that comprises a cleavage site for said particular protease. In such host cells, peptides may be identified that inhibit the protease, which results in increased stability of the biosensor and, consequently, in a modified expression of a reporter gene.

The method according to the invention may be used in the optimization of therapeutic peptides that have been previously described in the art. That is, a DNA library encoding a large number of sequence variants may be designed based on the sequence of a known therapeutic peptide. Various methods to introduce sequence variations into a template have been described in the art. Such DNA libraries may be used in the method according to the invention to identify peptide candidates with a higher biological activity.

In other embodiments, the DNA library may comprise computationally designed peptide candidates (for example peptides that have been identified in *in silico* docking studies) and sequence variants thereof. For example, a peptide that specifically binds to the active site of an enzyme or to the interaction site of two proteins may be designed computationally based on the three-dimensional structures of the involved proteins. Thus, the method according to the invention may be used to test a large number of computationally designed peptide candidates in parallel. Computationally designed peptides that have been identified to bind to a particular target may further be optimized using the method according to the invention as described above.

In certain embodiments, the DNA library may comprise random peptides. These random peptides may comprise the full set of available amino acids or a predetermined set of amino acids with certain physicochemical properties.

**4.7 The inducible promoter**

Within the present invention, the polynucleotide encoding the peptide candidate or the complex of non-ribosomal peptide biosynthesis enzymes, which is comprised in the DNA library, is under control of an inducible promoter. This allows the collection, analysis and comparison of samples that have been collected before and after the expression of a peptide candidate.

As used herein, the term "promoter" is a DNA sequence which extends upstream from the transcription initiation site and is involved in binding of RNA polymerase. The term "inducible promoter" as used herein refers to a promoter that is transcriptionally active when bound to a regulator that activates transcription or when a regulator that represses transcription is absent. The inducible promoter is operatively linked to a target sequence.

The term "operably linked" as used herein refers to a DNA sequence and regulatory sequence(s) are connected in such a way as to permit gene expression when the appropriate molecules are bound to the regulatory sequences. When the inducible promoter is regulated by a repressor, gene expression may occur in the absence of a repressor. When the inducible promoter is regulated by an environmental condition, gene expression occurs by obtaining the inducing environmental condition (e.g. an increase in temperature activating a heat shock promoter). Preferably, the inducible promoter is a promoter that can be activated by adding a chemical compound, i.e. an inducer, to the cell culture medium.

The skilled person is aware of inducible promoters that can be used to drive expression in a suitable host cell. That is, inducible promoters are typically host cell specific. If the host cell is *E. coli,* an inducible pBAD promoter may be used to drive expression of a peptide candidate or a complex of non-ribosomal peptide biosynthesis enzymes in the presence of the inducer L-arabinose. However, various other inducible promoter systems have been disclosed for use different types of host cells, including bacteria, yeasts and mammalian cells.

## 5. Hypo-sensitization of host cells

In certain embodiments, specific binding of a peptide to a target molecule directly or indirectly inhibits proliferation of the host cell. In such embodiments, overexpressing a target molecule may allow to identify highly active peptide variants. For example, when testing DNA libraries that comprise a high number of target binding peptides, such as DNA libraries that have been designed for the optimization of a known target binding peptide, the sensitivity of the host cell to such peptides may be reduced by overexpressing the target molecule. Assuming that the host cells in a sample comprise similar peptide levels, increasing the amount of a target molecule in a host cell may allow identifying peptides that bind to the target molecule more efficiently.

In certain embodiments, a heterologous target molecule or a fragment thereof may be comprised in a biosensor. In such embodiments, separate expression of the target molecule in the host cell may reduce the sensitivity of the host cell for a target binding peptide. In such embodiments, a host cell may express two forms of a target molecule, a first form that is comprised in a biosensor and generates a measurable output signal upon binding by a peptide, and a second form, which is not comprised in a biosensor and merely serves the purpose of capturing target binding peptides, thereby reducing the concentration of unbound peptide in the host cell.

In certain embodiments, the target molecule is an endogenous target molecule. In such embodiments, overexpression of the target molecule results in higher levels of the target molecule in the host cell and, consequently, a higher number of target molecules have to be inactivated by a target-binding peptide to achieve a growth inhibiting effect. Thus, peptides that inhibit endogenous target molecules more efficiently may be distinguished from less efficient binders.

Within the present invention, a target molecule may be encoded in the DNA library. That is, the members of the DNA library may encode a) a peptide candidate or a candidate complex of non-ribosomal peptide biosynthesis enzymes, preferably under control of an inducible promoter, and b) a target molecule. In certain embodiments, all members of the library encode the same target molecule, while the peptide candidates or the complexes of non-ribosomal peptide biosynthesis enzymes encoded in the DNA library may differ between library members.

## 6. Hyper-sensitization of host cells

In certain embodiments, the sensitivity of a host cell for a target binding peptide may be increased, such that also weaker interactions between peptides and target molecules can be captured. For example, certain target molecules may be considered as undruggable. In such cases, hyper-sensitizing a host cell for peptides that bind to a target molecule that is considered undruggable may allow for the identification of peptides that bind to the target molecule with only low specificity. These weak binders may then later on be optimized using the method according to the invention.

Hyper-sensitization may be achieved by reducing the concentration of a target binding peptide in a host cell, such that the remaining target molecules can be inactivated also by weak binders.

If the target molecule is a heterologous target molecule comprised in a biosensor, lowering the concentration of said target molecule may be achieved by lowering the expression of the polynucleotide encoding the biosensor.

In embodiments where the target molecule is an endogenous target molecule, lowering the expression of the endogenous target molecule may include the use of small inhibitory RNAs.

The term "small RNA" as used herein refers to an antisense RNA that can interact with another RNA, preferably an mRNA. Preferably, the small RNA binds to an mRNA such that it inhibits translation of the mRNA at least to a certain degree. Small RNAs that inhibit translation of mRNAs have been described in various host cells, including prokaryotic and eukaryotic host cells. Small RNAs may be naturally occurring RNAs or may be artificial RNAs that have been designed based on the sequence of a particular mRNA.

In certain embodiments, hyper-sensitization of target cells may be used to identify peptides that bind to endogenous target molecules that are considered undruggable. By using a small inhibitory RNA that interacts with the mRNA of the target molecule, the target molecule may be decreased to a level that is just sufficient to support growth of the host cell. Expressing a DNA library encoding peptide candidates and/or candidate complexes of non-ribosomal peptide biosynthesis enzymes in these modified host cells may allow identifying peptides that are potent enough to inhibit the remaining target molecules in the host cell but would not be potent enough to inhibit wild type levels of the target molecule.

The screening of DNA libraries comprising more than one known antimicrobial peptide in hyper-sensitized host cells may require the parallel screening of the same or similar DNA library in a non-hyper-sensitized host cell. In such embodiments, an antimicrobial peptide would be expected to act significantly faster on a hyper-sensitized host cell in which the target molecule of said antimicrobial peptide is downregulated compared to the non-hyper-sensitized host cell. Thus, identification of antimicrobial peptide - target molecule pairs in hyper-sensitized host cells may, in certain embodiments comprise the parallel screening of a DNA library in non-hyper-sensitized host cells.

In certain embodiments, hyper-sensitization of host cells may be used when screening peptides that have been computationally designed specifically for the down-regulated target molecule.

Within the present invention, a small inhibitory RNA may be encoded in the DNA library. That is, the members of the DNA library may encode a) a peptide candidate or a candidate complex of non-ribosomal peptide biosynthesis enzymes, preferably under control of an inducible promoter, and b) a small inhibitory RNA. In certain embodiments, all members of the library encode the same small inhibitory RNA, while the peptide candidates or the complexes of non-ribosomal peptide biosynthesis enzymes encoded in the DNA library may differ between library members.

## 7. Simultaneous identification of target molecules and target binding peptides

In certain embodiments, the method according to the invention may be used to simultaneously identify target binding peptides and their endogenous target molecules. For that, a library of peptide candidates or candidate complexes of non-ribosomal peptide biosynthesis enzymes may be combined with a library of putative target molecules. Preferably, the two libraries are combined in a single DNA library. That is, each member of a DNA library may comprise a single polynucleotide that encodes a) a peptide candidate or a candidate complex of non-ribosomal biosynthesis enzymes, preferably under control of an inducible promoter, and b) a putative target molecule.

Host cells that have been transformed or transduced with such a library may be cultured under appropriate conditions and samples may be collected before and after inducing the expression of the peptide candidates and/or the complexes of non-ribosomal peptide biosynthesis enzymes. These samples may be analyzed by next generation sequencing and for at least one peptide candidate or at least one complex of non-ribosomal peptide biosynthesis enzymes, all putative target molecules that are co-encoded in library members encoding said peptide candidate or said complex of non-ribosomal peptide biosynthesis enzymes may be identified.

If library members encoding a particular peptide candidate or a particular complex of non-ribosomal peptide biosynthesis enzymes are abundant in a sample that has been taken after the induction step, and if said particular peptide candidate or said particular complex of non-ribosomal peptide biosynthesis enzymes is found in combination with various putative target molecules, said particular peptide candidate is unlikely to be a target binding peptide or said particular complex of non-ribosomal peptide biosynthesis enzymes is unlikely to synthesize a target binding peptide. If, however, library members encoding a particular peptide candidate or a particular complex of non-ribosomal peptide biosynthesis enzymes are predominantly found in combination with a single putative target molecule in a sample that has been taken after the induction step, said particular peptide candidate or said particular complex of non-ribosomal peptide biosynthesis enzymes is likely to be or synthesize a target binding peptide and the putative target molecule is likely to be the target molecule of said particular peptide or of a peptide synthesized by said particular complex of non-ribosomal peptide biosynthesis enzymes. For this, each member of a DNA library preferably encodes a peptide candidate or a complex of non-ribosomal peptide biosynthesis enzymes and a putative target molecule, such that (part of) the genetic information of the peptide candidate / complex of non-ribosomal peptide biosynthesis enzymes and (part of) the genetic information of the putative target molecule can be captured in a single sequencing read.

## EXAMPLES

High-throughput methods are fundamental for discovery and characterization of bioactive peptides. The inventors developed the method of the invention to address two major limitations in the field: peptide synthesis and functional analysis. Using the method of the invention, the activity of 10,663 naturally occurring peptides including nearly all currently known antimicrobial peptides was profiled. The method provides unparalleled insight into the functional diversity of antimicrobial peptides and delivers a large number of previously unknown antimicrobials.

For that, the inventors first designed a library of naturally-encoded peptides. For this, the inventors collected the amino acid sequences of 3,063 peptides with experimentally validated activity ("PARENTS" from here on) from the antimicrobial peptide database (APD) (FIG. 1). Notably, PARENTS differed considerably with respect to the host from which they were derived, length, physiochemical properties, chemical modifications, 3D-structure, and sequence. Next, the inventors applied *tblastn* on the translated nucleotide databases accessible through the NCBI using the amino acid sequence of the PARENTS as queries. This search yielded 36,898 amino acid sequences with a similarity of $\geq 21.1\%$ to the PARENTS ("SIMILARS" from here on). Unlike the PARENTS, few SIMILARS have been synthesized or experimentally tested. However, owing to their natural origin and similarity to the PARENTS, a fraction of the SIMILARS is likely to display antimicrobial properties. For technical reasons, the inventors applied a cut-off of peptide chain length of 42 amino acids and selected SIMILARS with at least 62.2% sequence similarity. In this way, a library of 2,122 PARENTS and 10,300 SIMILARS was obtained. Examination of the final library indicated net charges from - 10 to +15 and hydrophobicity of -3.5 to 2.9 (GRAVY scale; FIG.2). Additionally, the inventors were able to allocate the origin of 7,497 of these peptides to the kingdom animalia, 74 to fungi, 678 to bacteria, and 2,485 to plantae (FIG.3).

The inventors converted the peptides into corresponding oligonucleotides (FIG.4), retrieved the latter as a pool after chemical synthesis on a microarray, and ligated the sequences into a plasmid on which their expression was controlled by the tightly regulated PBAD promotor (FIG.5). The inventors then transformed the model organism, *E. coli* TOP10, with the peptide-encoding DNA library. Due to a high sequence bias in the initial oligonucleotide pool (FIG.6), the inventors only identified 10,663 different peptide-encoding DNA sequences in *E. coli* using next-generation sequencing (NGS).

In order to assess the antimicrobial activity of the DNA-encoded peptides, the inventors performed the method of the invention and generated growth curves for each of the 10,663 peptide-expressing *E. coli* strains. To do so, the inventors inoculated three liquid cultures each with 500 million transformed cells and induced peptide synthesis after four cell doublings (FIG.7). Because expression of an antimicrobial peptide should inhibit growth of the expressing host, the propagation rate of the peptide-encoding DNA will also be reduced. Hence, the inventors harvested bacteria at the time of induction as well as 1.5 h, 3.0 h and 4.5 h post induction and used NGS to count reads for each peptide-encoding DNA. To derive growth curves (FIG.8), the inventors calculated the abundance of each strain (ID) using the respective NGS read counts and multiplied these with the measured cell concentration of the entire liquid cultures (OD) thereby obtaining an approximation of the strain-specific concentrations ($OD_{ID}$) at each sampling point. Comparing $OD_{ID}$ of all peptide-expressing strains after 4.5 h, the inventors found that intracellular expression of 1,240 peptides (11.6%) significantly inhibited growth of their host ("method-actives" from here on; Wald's test, p-value (*p*)<0.05, adjusted for multiple testing (adj.); FIG.9). The remaining peptides did not show growth inhibition in the method of the invention ("method-negatives" from here on), likely because they are not antimicrobial at all or require chemical modifications not introduced in *E. coli,* could not access their (e.g. extracellular) target, or did not reach inhibitory concentrations due to limited mRNA or peptide stability.

Next, the inventors confirmed that the intracellularly synthesized peptides also inhibited growth if the strains were grown individually. For this, the inventors selected 110 peptide-expressing strains experiencing different levels of growth inhibition in the method of the invention and measured their growth in microtiter plate wells (FIG.10). As the growth curves recorded in the method of the invention and in microtiter plates were comparable (FIG.11), the inventors concluded that complex dynamic of the method did not bias the results.

Screening 10,663 peptides at once allowed the inventors to address a number of research questions. Firstly, the inventors sought to confirm that our approach of exploiting sequence similarities to known antimicrobial peptides indeed allowed the inventors to identify antimicrobials. In fact, 1,035 out of 1,240 method-actives (83 %) were SIMILARS, i.e. peptides whose functions were not reported on the APD. A closer look revealed that for 310 inactive PARENTS the inventors found at least one active SIMILAR. As an example, PARENT Apo5 APOC1$_{667\ APD}$ (nomenclature: name of PARENT on APD $_{ID}$ origin), itself inactive, yielded 36 SIMILARS of which one showed eight amino acid differences to the PARENT, and displayed antimicrobial activity (FIG.12 and FIG.13). The inventors argue that the amino acids by which the inactive PARENT and the active SIMILAR differed were of high importance for activity and necessary for evading above-mentioned reasons for failed growth inhibition in the method of the invention. Furthermore, 47 PARENTS spawned an overrepresentation of active SIMILARS (Fisher's exact test, adj. p<0.05; FIG.14). Examples include Myticin-B (21/31), which spawned 31 SIMILARS, of which 21 were active, and PepG1 (11/11). This indicates that the respective peptide sequences have considerable plasticity and can accommodate multiple amino acids exchanges without losing activity. The inventors argue that these peptides might be well suited for additional modifications performed for instance in the course of lead optimization programs.

Secondly, the inventors evaluated the phylogeny of the hosts from which the inhibitory peptides were derived. For this, all peptides of the library were grouped taxonomically based on their natural host. The inventors then calculated the fraction (%) of method-actives within the ranks Kingdom and Class (Fisher's exact test; FIG.15). Method-actives were significantly underrepresented (p<0.05) among bacteria (8.5%), amphibians (7.7%), and mammals (10.3%) but overrepresented (p<0.05) in insects (13.4%), birds (25%), ray-finned fishes (15.6%) and bivalves (31.8%). Since insects contain by far the most species in the animal kingdom, this indicates a huge and so far undiscovered pool of antimicrobials in insects.

Thirdly, as cationic and hydrophobic peptides generally display antimicrobial activity, the inventors wondered whether growth inhibition the method of the invention was biased by the physiochemical properties of peptides. However, linear regression analysis indicated no correlation of growth inhibition with hydrophobicity (correlation = 0.04) and charge (correlation = -0.01; FIG.16). Furthermore, among the 47 PARENTS with overrepresented active SIMILARS, there was no clear relationship between charge or hydrophobicity and growth inhibition (FIG.17). The inventors thus conclude that growth inhibition in method-actives is driven by the specific antimicrobial activity of a peptide either damaging the cytoplasmic membrane or binding and inhibiting other cellular components.

Lastly, the inventors characterized the 50 most growth inhibitory (38 SIMILARS, 12 PARENTS) peptides using different assays (FIG.18). The inventors first used two biosensor constructs, containing the cspA and recA promoters, which upon activation are indicative of translation impairment and DNA damage, respectively. The results indicated translational impairment for 11 and DNA damage for 12 peptide-expressing strains (one sided t-test, adj. p<0.05; FIG.19), which suggests intracellular targets for these peptides. For example, Metalnikowin IIA$_{8984\ APD}$, Metalnikowin III$_{9011\ APD}$, known ribosomal inhibitors, and Pyrrhocoricin$_{7122\ NCBI}$, whose PARENT is also a ribosomal inhibitor, caused the strongest indication for translational impairment. Next, the inventors measured membrane damage by quantifying propidium iodide (PI) uptake.

Expression of 11 peptides resulted in membrane damage, with strongest damages observed for Delta Lysin I SIMILARS whose PARENT is a membrane pore inducing bacteriocin from *Staphylococcus*. Interestingly, all 11 peptides that caused membrane damage significantly inhibited growth already after 1.5 h in the method of the invention (Wald's test, p<0.05) (FIG.20) while for 25 peptides, and especially for those with putative intracellular targets, growth inhibition started only after 4.5 h (FIG.20). Noteworthy, delay of the growth inhibition onset is indicative for peptides that interact with an intracellular target. The inventors concluded that this effect could be observed with the method of the invention and hence reanalyzed all growth curves recorded for the method-actives. Growth was significantly inhibited after 1.5 h for 806 peptides (65%) suggesting membrane damage but only after 4.5 h in the case of the remaining 434 peptides, suggesting interaction with an intracellular target. As transition from the discovery pipeline to the patient is often hampered by general toxicity of membrane damaging peptides, the method of the invention could hence be a valuable tool for the high-throughput discovery of antimicrobial peptides that do not predominantly rely on this mechanism.

Finally, the inventors chemically synthesized 15 out of the 20 most growth 136 inhibitory peptides in the method of the invention and determined their minimal inhibitory concentrations (MIC; FIG.21) for *E. coli* TOP10. For five, no MIC was obtained; however, as four of these five were either PARENTS or derived from PARENTS known to not deliver a MIC with *E. coli* (Table 1), the inventors believe that these peptides exerted activity in the cytosol (directly at the place of their synthesis) but could not reach their target when applied externally. Remarkably, ten of the 15 peptides for which MICs were recorded, very efficiently inhibited growth of *E. coli* (MICs: 0.4 - 20 μM; mean = 3.7 μM; median = 1 μM), a concentration range that qualifies as a starting point for drug development. These results indicate that even though synthesized cytosolically, method-active peptides also strongly inhibited growth when added to cells externally. Additionally, the inventors selected the most active SIMILAR HFIAP-1$_{4545\ NCBI}$ and measured activity against other clinically relevant Gram negative and positive bacteria. SIMILAR HFIAP-1$_{4545\ NCBI}$ inhibited growth of these strains (MICs: 0.4 - 5.6 μM; FIG.21), which suggests a broad activity spectrum even though the method of the invention had been performed using *E. coli*.

Taken together, the method of the invention is suitable for the rapid discovery of naturally-occurring, and functionally diverse antimicrobial peptides. The inventors argue that the method will enable *de novo* design or optimization of peptides by directed evolution approaches and the inventors envision its application also in drug resistant (e.g. *Pseudomonas aeruginosa* or *Acinetobacter baumannii*) or recombinant strains used as reporters for microbial models relevant to human diseases.

Table 1:

| Rank (by OD ID 4.5h) | PARENT Name | ID | Sequence | Origin | Previously reported activity against *E. coli* |
|---|---|---|---|---|---|
| 1 | Meucin-25 | 11598 | VKLIQIRIWIQYVTVLQMFSMKTKQ | APD | not active |
| 2 | Ascaphin-6 | 9286 | GFKDWIKGAAKKLIKTVASSIANE | APD | active |
| 3 | P-10 | 8942 | VSKIKKYLKYKDRI | APD | active |
| 4 | Enterocin RJ-11 | 3780 | AIAKLVAKFGWPIVKKYYKQIMQFIGEGWAINKIIEWIKK | NCBI | Not determined |
| 5 | PepG1 | 11834 | MITISTMLQFGLFLIALIGLVIKLIELSIKK | NCBI | not active |
| 6 | PepG1 | 11828 | LVTLSAMLQFGIFLIAFIGLVIDLIKLSQKK | NCBI | not active |
| 7 | YFGAP | 8112 | VKVGINGFGRIGRLVTRAAFQSKKVEIVAIND | NCBI | active |
| 8 | PepG1 | 11833 | MITISTMLQFGLFLIALIGLVIKLIELSNK | NCBI | not active |
| 9 | YFGAP | 8135 | VKVGVNGFGRIGRLVTRAAFNSG KVEIVAIND | NCBI | active |
| 10 | Pyrrhocoricin | 7122 | VDKGGYLPRPTPPRPVYR | NCBI | active |
| 11 | Latarcin 4a | 5147 | LKDKVKSMGEKLKQYIQTWKAKF | APD | active |
| 12 | PepG1 | 11836 | LVYISIMLQFGMFIIAFIGLVIELIKLRQK | NCBI | not active |
| 13 | Vv-AMP1 | 8053 | RACESQSHRFKGTCVRQSNCAAVCQTE | NCBI | not active |
| 14 | BF-CATH | 9639 | KRFKKFFKKLKKSVKKRAKKFFKKPRVIGVSIPF | APD | active |
| 15 | Delta Lysine 1 | 3458 | MAADIISTIGDLVKWIIDTVNKFK | NCBI | not active |
| 16 | Maximin 3 | 5468 | TALKGAAKELASTYQH | NCBI | active |
| 17 | PepG1 | 11827 | PMLQFGLFLIALIGLVIKLIELSNKK | NCBI | not active |
| 18 | Oxyopinin 2b | 9690 | GKFSGFAKILKSIAKFFKGVGKVRKGFKEASDLDKNQ | APD | active |
| 19 | Cycloviolacin H2 | 10889 | SYIPCGESCVYIPCTVTALLGCSCSNKVCYKN | NCBI | not active |
| 20 | HFIAP-1 | 4545 | GWFKKAWRKVKHAGRRVLDTAKGVGRHYLNNWLNRYR | NCBI | active |

**Methods**

**Chemicals and reagents**

Unless otherwise stated, all chemicals, reagents and primers were obtained from Sigma Aldrich (Buchs, CH). Restriction enzymes and their buffers were obtained from New England Biolabs (Ipswich, USA). Synthetic genes were obtained from Integrated DNA Technologies (Leuven, BE) or Twist Bioscience (San Francisco, USA). Kits for the isolation of plasmid isolation and DNA purification kits were obtained from Zymo Research (Irvine, USA). Peptides in either purified (>90%) or crude format were obtained from Pepscan (Lelystad, NL). Sanger-sequencing was done at Microsynth (Balgach, CH).

**Bacterial strains and cultivations**

Unless otherwise stated, all experiments were performed using *Escherichia coli* TOP10 (F-*mcrA* Δ(*mrr-hsd*RMS-*mcr*BC) φ80/*acZ*ΔM15 Δ/*ac*X74 *rec*A1 oroD139 Δ(*ara-leu*)7697 *gal*U *gal*K λ—225 rpsL(StrR) *end*A1 *nup*G; Thermo Fisher Scientific, Waltham, USA). In this study, all cultivations were performed either in 14 ml polypropylene tubes (Greiner, Kremsmuenster, AT), filled with 5 ml of lysogeny broth (LB) medium (Difco, Becton Dickinson, Franklin Lakes, USA), or in 96-deepwell polypropylene plates (Greiner, Kremsmuenster, AT) filled with 500 μl of LB-medium. All samples were incubated at 37°C with agitation on a shaker (Kuhner, Birsfelden, CH) operated at 200 r.p.m. and 25 mm amplitude. All media were supplemented with the appropriate antibiotic for plasmid maintenance (50 μg ml$^{-1}$ kanamycin; 100 μg ml$^{-1}$ carbenicillin) and 1 % (w/v) D-glucose for repression of gene expression from catabolite-repression sensitive promoters such as PBAD. In case of peptide expression experiments, cultures were incubated without D-glucose and 0.3 % (w/v) of the inducer L-arabinose was used for induction. For all cultivations on solid medium, 15 mg ml$^{-1}$ agar (Difco) was added to the broth and incubation was performed without shaking in an incubator (Kuhner) at 37°C. If not indicated differently, the optical densities (OD) of bacterial cultures were determined by measuring light scattering at 600 nm using a UV/VIS spectrophotometer (Eppendorf, Hamburg, DE).

**In silico generation of peptide library**

The inventors collected all peptide sequences (called "PARENTS") available on the 'Antimicrobial Peptide Database' (APD) in May 2017 (http://aps.unmc.edu/AP/main.php, or https://wangapd3.com/main.php). These sequences were used as input queries to find sequence similar peptide sequences in the NCBI non-redundant nucleotide collection (nr/nt), a collection that holds sequences from GenBank, European Molecular Biology Laboratory (EMBL), DNA Databank of Japan (DDBJ) and Reference Sequence database (RefSeq), as well as translated protein information from the protein database (PDB). By applying *tblastn*, 170,300 additional peptide sequences (called SIMILARS) were found. Because the inventors were limited to 12,412 different peptides with a maximum length of 42 amino acids (the chosen platform for the synthesis of the peptide-encoding oligonucleotides allowed 12'412 different sequences with a maximal length of 170 bases), the inventors discarded SIMILARS with a sequence similarity to the respective parent of less than 62.2%. The following parameters were used for the *tblastn* search: maximum sequences = 100; matrix = BLOSUM62; gap cost = 11.1; word size = 6; active low complexity filter; adjustment = conditional compositional score matrix adjustment.

**Sequence distance among PARENTS and SIMILAR**

In order to visualize sequence diversity among PARENTS, the inventors created a sequence-based phylogenetic tree. The inventors performed pairwise global alignment of all PARENT sequences using the Needleman-Wunsch algorithm, as implemented in the R Bioconductor package 'Biostrings' (https://bioconductor.org/packages/release/bioc/html/Biostrings.html). The BLOSUM62 substitution matrix was used to compute the alignment scores, which were converted into pairwise distances following the method Scoredist. Based on the pairwise distances between PARENTS, the inventors used hierarchical clustering with average linkage to compute a dendrogram of sequences reflecting their similarities. PARENTS and their *tblastn*-derived SIMILARS were consolidated into groups, which were named after the PARENT from the APD (http://aps.unmc.edu/AP/main.php, or https://wangapd3.com/main.php). In the sequence-based phylogenetic tree, each SIMILAR was stacked on top of its PARENT at the tip of the dendrogram. A SIMILAR may appear multiple times if it was found multiple times in the *tblastn* search using different PARENTS.

**Peptide-encoding DNA architecture**

The corresponding oligonucleotide sequences of the peptide library were synthesized using microarray technology supplied from CustomArray Inc. (now GeneString, Piscataway, USA). The chosen platform allowed 12'412 different oligonucleotides with a maximal length of 170 bases. A generic oligonucleotide design employing four functional units was created (FIG.4): A coding unit, a filler unit, and two universal units for amplification. This process was automated for each sequence by using an in-house written script in R. The coding unit contained the reverse translation of the peptide amino acid sequence into a codon-optimized DNA for *E. coli*. The inventors always chose the most abundant codon for each amino acid. In cases in which restriction sites had been introduced that could potentially interfere with subsequent manipulations, the crucial codon was replaced by the second most abundant one for this amino acid. The filler sequence was added in order to compensate for the various lengths of peptide genes (shortest coding sequence = 15 nucleotides, longest coding sequence = 126 nucleotides) and adjust the total of filler and coding unit to 129 nucleotides for all members of the library. To do so, the inventors first added a UAA stop codon to the end of the coding sequence and then added downstream a semi-random sequence, ensuring a GC content of 40% for the filler sequence and limiting the number of identical nucleotides following each other to three. By adding this filler sequence the inventors maximized sequence disparity at DNA level (many coding sequences are homologs) thereby potentially increasing both synthesis and, later, sequencing quality. Two amplification units, of 23 and 18 bases, respectively, were appended upstream and downstream of the coding sequence and filler unit and contained the ribosomal binding site and restriction sites for the enzymes PstI and HindIII. Two amplify the peptide-encoding DNA, primer 1: CTGCACAAAGCTTACGTG, complementary to the upstream amplification unit and primer 2: CACGTAAGCTTTGTGCAG, reverse complementary to the downstream amplification unit were used.

**Peptide-encoding DNA cloning**

The chemically synthesized and single stranded oligonucleotides were separated from their array and the inventors received them as a pool. This pool was aliquoted in 10 mM Tris-HCl, 1 mM EDTA, pH 8 and deep-frozen at -80°C. The pool was amplified by polymerase chain reaction (PCR) in a 50 µl reaction using 5 ng of template and 10 µM HPLC-purified primer 1 and primer 2, complementary to the amplification sites, and 25 µl of Phusion® High-Fidelity PCR Master Mix with HF buffer. The amplification was performed using 25 cycles of 98°C for 15 s, 55°C for 20 s, and 72°C for 5 s. The now double stranded peptide-encoding DNA sequences were purified using a DNA purification kit. DNA concentration was measured using a NanoDrop 2000 Spectrophotometer (Thermo Fisher Scientific) and 500 ng of purified product was used for a restriction digest using enzymes HindIII-HF and PstI-HF in Cutsmart buffer. The digested product was again purified using a DNA purification kit and ligated to plasmid pBAD (Thermo Fisher Scientific) digested with the same enzymes. This plasmid harbored the tightly controllable PBAD promoter for peptide gene expression, a pBR322 replication of origin and a resistance gene encoding for beta-lactamase. For ligation, pBAD was purified using a 1% agarose gel and a DNA gel recovery kit after digestion. Next, T4 ligase (800 units) was used to ligate 100 ng of cut pBAD vector and 10 ng peptide encoding DNA sequences in T4 ligase buffer (molar ratio of 7:1 insert:vector). The ligation mix was incubated for 14 h at 16°C. The ligation product was dialysed in deionized water using filters (Milipore, Burlington, USA) and 1 µl of the mix was used to transform 20 µl of CloneCatcher™ Gold DH5G Electrocompetent *E. coli* (Genlantis, Burlington, USA) cells using electroporation. Recovered cells were plated and incubated overnight an LB agar plates supplemented with carbenicillin. Afterwards, ~500,000 colonies were washed off the plates using LB medium and the plasmids containing the peptide-encoding DNA sequences were extracted from 2.5*109 cells using a plasmid isolation kit. An aliquot of 5 ng of these plasmids was used to transform *E. coli* TOP10 cells using the protocol from the transformation above. A total of 1'000'000 colonies were recovered from the plates after overnight incubation by washing with LB medium, the suspension was diluted to OD = 1 with LB-medium, glycerol was added to a final concentration of 20% (v/v), and aliquots of 500 million cells were stored at -80°C.

**Growth experiment**

Three aliquots of *E. coli* TOP10 harboring the peptide-encoding DNA sequences on the pBAD plasmid were thawed and added to three 1 l baffled shake flasks containing 100 ml of LB medium + 100 µg ml$^{-1}$ carbenicillin. The cultures were grown for roughly 7.5 h at 37°C. When the OD reached 0.2, the cultures were supplemented with L-arabinose to a final concentration of 0.3 % (w/v) to induce peptide expression. Cell samples were taken from each biological replicate at the point of induction and 1.5 h, 3 h, and 4.5 h post induction. The plasmids were extracted from all samples using a plasmid isolation kit.

**Next-generation sequencing (NGS)**

For the generation of growth curves according to the method of the invention, peptide-encoding DNA sequences on plasmids, collected from the three replicates across four time points during the growth experiment, were sequenced by NGS. Additionally, the abundance of peptide-encoding DNA sequences in the original oligonucleotide pool and after transformation of the assay strain *E. coli* TOP10 was assessed by NGS as well. Peptide-encoding DNA sequences were amplified by primer 1 and primer 2 using 100 ng of plasmid and the PCR-amplification protocol mentioned before, but only for 10 cycles to avoid amplification bias. The amplification product was purified using an agarose gel. Single Index PentAdapters from Pentabase were used to prepare PCR-free libraries with the KAPA HyperPrep Kit (now Roche, Basel, CH) according to manufacturer's specifications. Libraries were quantified using the qPCR KAPA Library Quantification Kit. Libraries were pooled and sequenced PE 2x151 with an Illumina HiSeq 2500 using v4 SBS chemistry. Roughly 10% genomic PhiX library as spike in to increase sequence diversity. Basecalling was done with bcl2fastq v2.20.0.422. The resulting fastq files were processed using an in-house software written in R and C. This software aligns each sequence to our reference table of 12'412 sequences linking peptide-encoding DNA sequences and peptide sequence, identifies mismatches and sequencing errors, and counts how often each peptide-encoding DNA sequences was sequenced in each sample (= NGS read counts).

**Generation of growth curves with the method of the invention**

The inventors used the standard workflow of DESeq2 (NGS read count normalization, dispersion estimates, and Wald's tests) to analyze NGS read counts. Only sequences that passed independent filtering were included further analyses (= 10,633). To draw growth curves for each peptide-expressing strain, the inventors calculated the log2-fold changes of NGS read counts between the time of induction and all other time points (1.5 h, 3.0 h and 4.5 h post induction). A Bayesian shrinkage estimator was employed to shrink the log2 fold-change for each ID (IfcShrinksID) between all time points using the R/Bioconductor package 'apeglm'. To draw the growth curves, the inventors calculated a strain specific $OD_{ID}$ at each time point according to equation (1). OD values at the specific time points were averaged values from all three biological replicates (FIG.7). The $OD_{ID}$ (0 h) for each peptide-expressing strain was set to 0.2 at the time of induction as IfcShrinkID (0 h) = 0 and OD = 0.2. This enabled the inventors to compare peptide-expressing strains of different abundancies (see FIG.6). $OD_{ID}$ values can be interpreted as the OD values that would have been measured when incubating the respective strain individually in the same experiment, i.e. in this case in LB medium in a 100 ml shake flasks.

$$(1)\ OD_{ID}(t) = OD(t) \times 2^{IfcShrinkID(t)}$$

To find method-active peptides, the inventors also performed a one-sided Wald's tests, with the alternative hypothesis that the expression of a given peptide leads to a reduced $OD_{ID}$ 1.5 h and 4.5 h post induction. The inventors rejected the null hypothesis at significance level alpha = 0.05. Peptides with a $p<0.05$ (after adjustment for multiple testing using the Benjamini-Hochberg method) after 4.5 h are considered method-active peptides. Peptides with $p<0.05$ after 1.5 h do significantly inhibit growth already after 1.5 h.

**Monoseptic growth experiments**

Taking the $OD_{ID}$ (4.5 h) of each peptide-expressing strain, the inventors could rank all peptides by their growth inhibitory effect. The inventors selected 110 peptides (Ranks 1-50, 100-119, 1000-1019, and 10,000-10,019) and then generated an identical copy of the strain previously used in the method of the invention for its expression. First, the corresponding peptide-encoding DNA-sequences were synthesized as gene fragments. An aliquot of 400 ng of each gene fragment was directly used for a restriction digest using enzymes HindIII-HF and Pst-HF in Cutsmart buffer. The product was purified using a DNA purification kit. Next, T4 ligase (800 units) was used to ligate 50 ng of identically digested pBAD vector and 10 ng of purified gene fragment in T4 ligase buffer for 14 h at 16°C. The ligation product was purified using a DNA purification kit. An aliquot of 5 μl of the purified ligation product were then used to transform chemically competent *E. coli* TOP10 cells. From the resulting colonies, the inventors isolated one strain, sequence-verified the correct assembly of the expression plasmid, and stored it after overnight growth in glycerol at -80°C. For the growth experiment, the inventors first re-isolated single colonies on solid media and then picked three clones, incubated them separately overnight and inoculated into 200 μl fresh LB medium containing 0.3 % (w/v) L-arabinose to a final OD of 0.01 into 96-well micro titer plates (Greiner). Growth was recorded by measuring OD in a Tecan Infinite 200 PRO (Tecan, Männedorf, CH) for 4.5 h (37°C, 1.5 mm orbital shaking).

**Enrichment analyses**

The inventors used Fisher's exact test in order to assess over- or underrepresentation of method-actives in various groups. This amounts to a hypergeometric test to assess the significance of drawing n active peptides in a group of k, from a population of size *N* containing *K* active peptides. The inventors rejected the null hypothesis at significance level alpha = 0.05. Groups with a $p<0.05$ had a significantly different representation of active peptides compared with the overall population. When adjusting for multiple testing, the inventors used the Benjamini-Hochberg method.

**Peptide classifications**

The physiochemical parameters of the peptides were calculated at pH 7 using the R package 'Peptides' (https://cran.r-project.org/package=Peptides). For charge, the inventors used the method by Lehninger (Butterworth, P.J. Lehninger: principles of biochemistry (4th edn) D.L. Nelson and M.C. Cox, W.H. Freeman % Co., New York, 1119pp (plus 17 pp glossary). ISBN 0-7167-4339-6 (2004). Cell Biochem. Funct. 23, 293-294 (2005)). For hydrophobicity, the inventors used the calculations by KyteDoolittle Kyte, J. / Doolittle, R.F. A simple method for displaying the hydrophatic character of a protein. J Mol. Biol. 157, 2015-132 (1982)). The information for each PARENT such as the name, chemical modification, activity, 3D-structure, was extracted from the APD website (http://aps.unmc.edu/AP/main.php, or https://wangapd3.com/main.php) using an in-house R script. The information on the species from which a specific peptide sequence originated, was extracted from the *tblastn* search and from the APD website. The entire taxonomic classifications (kingdom, phylum, class) for each species were extracted, if available, from the Global Biodiversity Information Facility Data Portal (https://gbif.org) using the R package 'taxize' (https://cran.r-project.org/package=taxize).

**Purification of chemically synthesized peptides**

Peptides were obtained from Pepscan (Lelystad, NL) in >90% purity or in crude format and subsequently purified to >90% purity in house. For the latter, crude peptides were dissolved in 5 ml DMSO and 15 ml 0.1% aqueous trifluoroacetic acid, TFA. HPLC purification of the dissolved crude peptides was performed on an ÄKTAexplorer chromatography system (GE Heathcare, SE). The entire peptide sample was loaded onto a RP C18 column (PRONTOSIL 120 C18 10 $\mu$m, 250 x 20 mm, 50 x 20 mm precolumn, Bischoff, Leonberg, DE), heated to 30°C and operated at a flow rate of 10 ml min$^{-1}$ using 0.1% aqueous TFA as solvent A and acetonitrile supplemented with 0.1% TFA as solvent B. The ratios of A to B were adapted for each peptide and typical values are given below. The column was equilibrated with the peptide-specific mixture of solvent A and solvent B 0-20%) prior to injection. After injection and an initial wash step of 6 min a gradient was imposed with the same mixture, and then a gradient was applied, in the course of which the amount of solvent B was increased to 50-90 % in 40 min. The column was washed with 95 % solvent B for 8 min and equilibrated with the specific solvent A/solvent B mixture for the next run for 13 min. Peptide elution was monitored spectrophotometrically at 205 nm and generally the main peptide peak was collected. The sample was frozen at -80 °C for >2 h and lyophilized (approx. 18 h) using a freeze-dryer (Alpha 2-4 LDplus, Christ, DE), connected to a vacuum pump (RC6, Vacuubrand, DE). The lyophilized peptides were dissolved in 1 ml DMSO and stored at -20 °C. The concentration of the peptide stocks was determined via HPLC using an Agilent 1200 series HPLC system. Each peptide stock was analysed as a 1:100 dilution in water. An aliquot of 10 $\mu$l of the peptide stock was injected onto an RP-C18 column (ReproSil-Pur Basic C18, 50 x 3 mm, Dr. Maisch, Germany) operated with water supplemented with 0.1 % TFA as solvent A and acetonitrile supplemented with 0.1 % TFA as solvent B. Separation was performed using the same concentration profile previously used for purification. The concentration was measured using the integrated peak area at 205 nm and then calculated using peptide-specific absorption properties.

**Measurement of the minimal inhibitory concentration (MIC)**

On the same day at which MIC assays were executed, purified peptides were thawed and the concentration was determined by HPLC as described before. *E.coli* TOP10 cells were grown in Mueller Hinton Broth (MHB) or diluted MHB (25 % of the original strength) over night to stationary phase. Diluted MHB has been frequently used to assay antimicrobial peptides 12. The cultures were then supplemented with 20 % glycerol, aliquoted and frozen at -80°C. For MIC measurements, a frozen stock of the cells was thawed, resuspended in MHB or 25% MHB to adjust to a density of $5 \cdot 10^5$ cells ml$^{-1}$ in the experiment, and distributed to microtiterplate wells by an automated liquid handling system (Hamilton, Bonaduz, CH). Then the peptides were added by the liquid handling system in 2-fold dilutions using minimum 100 $\mu$g ml$^{-1}$ as the highest concentration. MICs were determined as broth microdilution assay in 384-well flat bottom polypropylene plates (Falcon® 96-Well Flat-Bottom Microplate) adapted from the protocol of Wiegand et al. The plates were sealed airtight and incubated for 18 h without shaking at 37°C before reading the OD using a Tecan Infinite 200 PRO plate reader. The MIC value corresponded to the concentration at which no growth of the bacterial strain was observed (< 5% of the OD value of the growth control). MIC experiments were performed at least in triplicate.

**Membrane damage assay**

The inventors selected the peptide-expressing strains of rank 1-50 in the method of the invention that the inventors had previously constructed for the monoseptic growth assay. Additionally, the inventors selected the strain expressing the inactive control peptide HNP-13425 APD, a peptide known to be inactive if expressed in *E.coli*. Each strain was re-isolated on solid media from a frozen stock and incubated overnight. Then, two colonies were picked and incubated overnight in 96-deepwell polypropylene plates. These cultures were used to inoculate fresh media containing 0.3 % (w/v) L-arabinose to a final OD of 0.01 into 96-well microtiter plates. The plates were then incubated on for 4.5 h (37 °C, 1.5 mm orbital shaking). After 4.5 h, an aliquot of 50 $\mu$l of cell suspension a Tecan Infinite 200 PRO plate reader was added to 150 $\mu$l of phosphate-buffered saline into a fresh 96-well microtiter plate. Propidium iodide (PI) was added to a final concentration of 1 $\mu$g ml$^{-1}$. PI is a DNA-intercalating dye which cannot pass an intact cytoplasmic membrane. For each sample, PI fluorescence ($\lambda$Ex= 579 nm / $\lambda$Em= 616 nm) of ~10,000 cells was analysed using a flow cytometer LSR Fortessa (BD Biosciences, Allschwil, CH). To determine the membrane damaging properties of each of the expressed peptides, the inventors calculated the fraction of cells in percent for which a PI uptake was measured using the software FlowJo V10 (BD Biosciences).

**Stress response assay**

The inventors selected peptide-expressing strains of rank 1-50, previously generated for the monoseptic growth assay. Additionally, the inventors selected the strain expressing the inactive control peptide HNP-1$_{3425\text{ APD}}$. Moreover, two plasmids (cloning vector: puA66) containing either the promoter of the gene for recombinase A (PrecA) or for the gene for cold shock protein A (PcspA) were purified from the *E. coli* Alon collection. Both plasmids contained a transcriptional fusion of their promoter with a downstream gene for green fluorescent protein (*gfp*), an additional kanamycin resistance cassette and a pSC101 origin of replication. The inventors transformed each of the 51 peptide-expressing *E. coli* strains with each of the two plasmids to generate 102 different strains and incubated them overnight on solid media. Then, three colonies were picked and incubated overnight. These cultures were used to inoculate fresh media containing 0.3 % (w/v) L-arabinose to a final OD of 0.05 into 96-well microtiter plates. The inventors recorded OD and GFP expression ($\lambda$Ex 488 nm/$\lambda$Em 530nm) after 1.5 h and 4.5 h using a Tecan Infinite 200 PRO (37 °C, 1.5 mm orbital shaking). For each strain, the inventors calculated the specific fluorescence change between the two time points (GFP/OD (4.5 h) - GFP/OD (1.5 h)). Statistical significance was calculated by one sided t-tests, adjusted for multiple testing by Benjamini-Hochberg, using the signal of HNP-1$_{3425\text{ APD}}$

**Claims**

1. A method for identifying a peptide which binds specifically to a target molecule, the method comprising the steps of:

    (a) introducing a DNA library into a plurality of host cells, the DNA library comprising at least two members, wherein each of the at least two DNA library members comprises a nucleic acid sequence encoding

    (i) a peptide candidate; or
    (ii) a candidate complex of non-ribosomal peptide biosynthesis enzymes; wherein the nucleic acid sequence encoding the peptide candidate or the candidate complex of non-ribosomal peptide biosynthesis enzymes is operably linked to an inducible promoter;

    wherein the host cell comprises the target molecule, and wherein a measurable output signal is generated or inhibited upon specific binding of a peptide to said target molecule;
    (b) culturing the plurality of host cells obtained in step (a);
    (c) inducing the synthesis of the peptide candidates and/or the candidate complex of non-ribosomal peptide biosynthesis enzymes in the plurality of host cells cultured in step (b);
    (d) collecting a first sample at a first time point from the culture of step (b) before the induction step (c) and collecting a second sample at a second time point from the culture of step (b) after the induction step (c);
    (e) determining the abundance of measurable output signals in the samples taken at the first time point and at the second time point for at least one peptide candidate or at least one candidate complex of non-ribosomal peptide biosynthesis enzymes; and
    (f) identifying

    (i) a peptide candidate to be a target binding peptide if the abundance of measurable output signals generated by said peptide candidate is different between the first time point and the second time point; and/or
    (ii) a candidate complex of non-ribosomal peptide biosynthesis enzymes to synthesize a target binding peptide if the abundance of measurable output signals generated by said candidate complex of non-ribosomal peptide biosynthesis enzymes is different between the first time point and the second time point.

2. The method according to claim 1, wherein two or more samples are collected after the induction step (c), and wherein the steps (e) and (f) are performed with each sample.

3. The method according to claim 1 or 2, wherein the target molecule is

   i) an essential component of the host cell; or
   ii) a component of a cell, preferably a mammalian cell, in particular wherein said component is comprised in a recombinant biosensor.

4. The method according to any one of claims 1 to 3, wherein the measurable output signal corresponds to

   i) cell viability; or
   ii) expression of a reporter gene, in particular wherein the reporter gene is
   i) an antibiotic resistance gene;
   ii) an auxotrophic marker; or
   iii) a gene encoding a DNA-modifying enzyme.

5. The method according to any one of claims 1 to 4, wherein the peptide candidate or the peptide synthesized by the candidate complex of non-ribosomal peptide biosynthesis enzymes is a linear or a cyclic peptide.

6. The method according to any one of claims 1 to 5, wherein the DNA library encodes at least one peptide candidate and at least one candidate complex of non-ribosomal peptide biosynthesis enzymes.

7. The method according to any one of claims 1 to 6, wherein the host cell is a microbial host cell, in particular wherein the microbial host cell is a yeast cell or a bacterial cell, in particular wherein the bacterial cell is a pathogenic bacterium, in particular wherein the pathogenic bacterium is selected from the group consisting of: *Pseudomonas aeruginosa, Klebsiella pneumonia, Acinetobacter* spp., *Escherichia coli, Enterobacter* spp., *Staphylococcus aureus* and *Enterococcus* spp.

8. The method according to any one of claims 1 to 7, wherein the host cell is a protease deficient host cell and/or wherein the host cell comprises an enzyme that catalyses the cyclization of a candidate peptide.

9. The method according to any one of claims 1 to 8, wherein the abundance of measurable output signals in a sample is determined by sequencing, in particular by next-generation sequencing.

10. The method according to any one of claims 1 to 9, the method comprising an additional step of overexpressing a target molecule in the plurality of host cells, in particular wherein the DNA library comprises a nucleic acid molecule encoding a target molecule, preferably wherein each member of the DNA library encodes

    i) a peptide candidate or a candidate complex of complex of non-ribosomal peptide biosynthesis enzymes; and
    ii) a target molecule.

11. The method according to any one of claims 1 to 9, the method comprising an additional step of expressing a small inhibitory RNA in the plurality of host cells, in particular wherein the small inhibitory RNA inhibits the synthesis of a target molecule, in particular wherein the DNA library comprises a nucleic acid molecule encoding a small inhibitory RNA, preferably wherein each member of the DNA library encodes

    i) a peptide candidate or a candidate complex of complex of non-ribosomal peptide biosynthesis enzymes; and
    ii) a small inhibitory RNA.

12. A DNA library comprising at least two plasmids, wherein each of the at least two plasmids comprises a nucleotide sequence encoding

    (a) a peptide candidate or a candidate complex of non-ribosomal peptide biosynthesis enzymes; and
    (b) a target molecule or an enzyme that is involved in the biosynthesis of a target molecule.

13. The DNA library according to claim 12, wherein in each of the at least two plasmids, the nucleotide sequence encoding the peptide candidate or the candidate complex of non-ribosomal peptide biosynthesis enzymes is operably linked to a first promoter sequence and wherein the nucleotide sequence encoding the target molecule or the enzyme that is involved in the biosynthesis of a target molecule is operably linked to a second promoter sequence.

14. The DNA library according to claim 12 or 13, wherein the target molecule is a protein originating from a microbial host cell, in particular from a pathogenic microbial host cell or wherein the target molecule is a protein that is involved in a human disease.

15. A DNA library comprising at least two plasmids, wherein each of the at least two plasmids comprises a nucleotide sequence encoding

(a) a peptide candidate or a candidate complex of non-ribosomal peptide biosynthesis enzymes; and
(b) a small inhibitory RNA; in particular

wherein in each of the at least two plasmids, the nucleotide sequence encoding the peptide candidate or the candidate complex of non-ribosomal peptide biosynthesis enzymes is operably linked to a first promoter sequence and wherein the nucleotide sequence encoding the small inhibitory RNA is operably linked to a second promoter sequence; and/or wherein the inhibitory small RNA inhibits synthesis of an essential protein from a microbial host organism, in particular from a pathogenic microbial host organism.

FIG.1

FIG. 2

FIG.3

FIG.4

FIG.5

FIG.6

FIG.7

FIG.8

FIG.9

FIG.10

FIG.11

$OD_{ID}$ (4.5 h)          OD (4.5 h)

FIG.12

| ID | | Class | Species |
|----|----|-------|---------|
| 677 | FST KTRNWFSEHFKKVKEKLKDTFA | Reptilia | *Alligator sinensis* |
| 9989 | ITT KTRNWFSEAFSKMKEKLQSAFS | Mammalia | *Ochotona princeps* |
| 691 | ..TKTRTWFSETFGKLKEKLKTTF. | Mammalia | *Mesocricetus auratus* |
| 695 | LSAKTRDWFSETFRKVKEKLK.... | Mammalia | *Pongo abelii* |
| 699 | ...KTRSWFSETLNKMKEKLKTTFA | Mammalia | *Rattus norvegicus* |
| 701 | LPAKTRDWFSETFRKVKEKLK.... | Mammalia | *Cercocebus atys* |
| 681 | ..TKARNWFSETYGKVKEKIKTTFA | Mammalia | *Nannospalax galili* |
| 697 | FSEKTQNWFSELFHKVKEKFETTF. | Mammalia | *Pteropus vampyrus* |
| 693 | ...KTRNWFSETFNKVKERFKTAFS | Mammalia | *Ailuropoda melanoleuca* |
| 702 | ..TKTRTVLSETFDKVKEKFKTTFA | Mammalia | *Peromyscus maniculatus* |
| 9988 | FATKTKNWFSEQYEKVKQKLSESF. | Actinopterygii | *Hemibarbus mylodon* |
| 704 | LSAKMREWFSETFQKVKEKLK.... | / | / |
| 9990 | .STKTRNWFSETLQKVKEKLK.... | Mammalia | *Carlito syrichta* |
| 709 | ....RAWFSEAFGKVKEKLKTTFS | / | / |
| 686 | ITTKTRNWFSETISKVKEKFKSTFS | Mammalia | *Oryctolagus cuniculus* |
| 700 | ..TKTRNWFSETFKKIKEKV.... | Amphibia | *Xenopus laevis* |
| 683 | ...KTRNWFSETFNKVKEQLKTAFS | Mammalia | *Ursus maritimus* |
| 706 | LSAKMRDWFSETFQKVKEKLK.... | Mammalia | *Gorilla gorilla* |
| 690 | ..TKTRAWFSETFGKVKEKLKTTFS | Mammalia | *Mus caroli* |
| 679 | ..TKTRAWFSEAFGKVKEKLKTTFS | Mammalia | *Mus musculus* |
| 9993 | IPTKTRNWFSETLQKVKEKL.... | Mammalia | *Callithrix jacchus* |
| 9991 | FPAKTRDWFSETFRKVKEKLK... | Mammalia | *Cebus capucinus* |
| 696 | IATKTRDWFSETFKKVKEK..... | Mammalia | *Papioanubis* |
| 703 | FPSKTRNWFSETYSKVKEKFKTTFS | Mammalia | *Propithecus coquereli* |
| 684 | ...KTRNWFSETFKKVKEQLKTAFS | Mammalia | *Rhinolophus sinicus* |
| 680 | ...KTRNWFSETFHKVKEQLKTAFS | Mammalia | *Odobenus rosmarus* |
| 694 | IPAKTRNWFSETFKKVKEQLKTAFS | Mammalia | *Leptonychotes weddellii* |
| 682 | IPAKTRNWFSETFHKVKEQLKTAFS | Mammalia | *Odobenus rosmarus* |
| 698 | IPAKTRNWFSETFHKVKEQLKTAFS | Mammalia | *Leptonychotes weddellii* |
| 692 | FSTKTRNWFKHNFQKVKDKMDATFS | Mammalia | *Phascolarctos cinereus* |
| 689 | FSEKTRNWFSEQFQKMKEKFNEQFS | Reptilia | *Chrysemys picta* |
| 685 | ...KTRNWFSETLNKMKEKLKTTFA | Mammalia | *Rattus norvegicus* |
| 687 | IPAKTRNWFSEAFKKVKEHLKTAFS | Mammalia | *Canis lupus* |
| 688 | ...KTRNWFSETFQKVKEKLK... | Mammalia | *Otolemur garnettii* |
| 707 | FADKTRHWLSETFKKIKEKLE... | Mammalia | *Galeopterus variegatus* |
| 708 | FPAKTWDWFSETFRKVKEKLK.... | Mammalia | *Mandrillus leucophaeus* |
| 678 | FATKTRTWFSETFTKVKEKLKDTFS | Mammalia | *Marmota marmota* |
| 9992 | IPAKTRNWFSETLQKVKEKLR... | Mammalia | *Aotus nancymaae* |

Legend:
- non conserved
- ≥ 50% conserved
- all match

FIG.13

FIG.14

FIG.15

FIG.16

FIG.17

FIG.18

FIG.19 continued

EP 4 112 728 A1

FIG.20

FiG.20 continued

Time [h]

FIG.21

| Rank | Peptide | Origin | ID | MIC [µM] in MHB I * | MIC [µM] in 25% MHB I * |
|---|---|---|---|---|---|
| 1 | Meucin-25 | APD | 11598 | >65 | >65 |
| 2 | Ascaphin-6 | APD | 9286 | 0.8 | 1 |
| 3 | P-10 | APD | 8942 | >70 | 13 |
| 4 | Enterocin RJ-11 | NCBI | 3780 | 0.5 | 1 |
| 5 | PepG1 | NCBI | 11834 | p.f. | p.f. |
| 6 | PepG1 | NCBI | 11828 | >72 | >72 |
| 7 | YFGAP | NCBI | 8112 | >60 | 8 |
| 8 | PepG1 | NCBI | 11833 | p.f. | p.f. |
| 9 | YFGAP | NCBI | 8135 | >60 | 15 |
| 10 | Pyrrhocoricin | NCBI | 7122 | 20 | 8 |
| 11 | Latarcin 4a | APD | 5147 | 8 | 5 |
| 12 | PepG1 | NCBI | 11836 | p.f | p.f. |
| 13 | Vv-AMP1 | NCBI | 8053 | >68 | >68 |
| 14 | BF-CATH | APD | 9639 | 1 | 1 |
| 15 | Delta Lysin 1 | NCBI | 3458 | >75 | >75 |
| 16 | Maximin 3 | NCBI | 5468 | >71 | >71 |
| 17 | PepG1 | NCBI | 11827 | p.f. | p.f. |
| 18 | Oxyopinin 2b | APD | 9690 | 0.5 | 0.5 |
| 19 | Cycloviolacin H2 | NCBI | 10889 | p.f. | p.f. |
| 20 | HFIAP-1 | NCBI | 4545 | 0.5 | 0.5 |

| HFIAP-1$_{4545\ NCBI}$ tested against clinical isolates | ATCC number | MIC [µM] in MHB I |
|---|---|---|
| Staphylococcus aureus | 29213 | 1.4 |
| Pseudomonas aeruginosa | 47085 | 0.7 |
| Enterococcus faecalis | 29212 | 5.6 |
| Klebsiella pneumonia | 13883 | 0.4 |
| Escherichia coli | 25922 | 0.7 |

Europäisches
Patentamt

European
Patent Office

Office européen
des brevets

# EUROPEAN SEARCH REPORT

**Application Number**

EP 21 18 2971

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BEHSAZ BAHAR ET AL: "Integrating genomics and metabolomics for scalable non-ribosomal peptide discovery", NATURE COMMUNICATIONS, vol. 12, no. 1, 28 May 2021 (2021-05-28), XP055871357, DOI: 10.1038/s41467-021-23502-4 Retrieved from the Internet: URL:https://www.nature.com/articles/s41467-021-23502-4.pdf> * see pages 3-12 * | 1-15 | INV. C12N15/10 C07K1/04 G01N33/68 |
| Y | M. WINN ET AL: "Recent advances in engineering nonribosomal peptide assembly lines", NATURAL PRODUCT REPORTS, vol. 33, no. 2, 1 January 2016 (2016-01-01), pages 317-347, XP055297369, GB ISSN: 0265-0568, DOI: 10.1039/C5NP00099H * the whole document * | 1-15 | |
| Y | RODERICH D. SÜSSMUTH ET AL: "Nonribosomal Peptide Synthesis-Principles and Prospects", ANGEWANDTE CHEMIE INTERNATIONAL EDITION, vol. 56, no. 14, 27 March 2017 (2017-03-27), pages 3770-3821, XP055487875, ISSN: 1433-7851, DOI: 10.1002/anie.201609079 * the whole document * | 1-15 | TECHNICAL FIELDS SEARCHED (IPC) C12N G01N C07K |

-/--

The present search report has been drawn up for all claims

| Place of search | Date of completion of the search | Examiner |
|---|---|---|
| Munich | 13 December 2021 | Vix, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 4**

Europäisches
Patentamt

European
Patent Office

Office européen
des brevets

# EUROPEAN SEARCH REPORT

**Application Number**

**EP 21 18 2971**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HUANG HSIN-MEI ET AL: "Unleashing the Potential of Ribosomal and Nonribosomal Peptide Biosynthesis", BIOCHEMISTRY, vol. 58, no. 2, 15 January 2019 (2019-01-15), pages 73-74, XP055871248, ISSN: 0006-2960, DOI: 10.1021/acs.biochem.8b00930 Retrieved from the Internet: URL:https://pubs.acs.org/doi/pdf/10.1021/acs.biochem.8b00930> * the whole document * | 1-15 | |
| Y | Martínez-núñez Mario Alberto ET AL: "Nonribosomal peptides synthetases andtheir applications inindustry", , 2 August 2016 (2016-08-02), pages 1-8, XP055871396, DOI: 10.1186/s40508-016-0057-6 Retrieved from the Internet: URL:https://sustainablechemicalprocesses.springeropen.com/track/pdf/10.1186/s40508-016-0057-6.pdf [retrieved on 2021-12-09] * the whole document * | 1-15 | TECHNICAL FIELDS SEARCHED (IPC) |
| Y | WO 2020/123387 A1 (LASSOGEN INC [US]) 18 June 2020 (2020-06-18) * see claims * | 1-15 | |

-/--

The present search report has been drawn up for all claims

| Place of search | Date of completion of the search | Examiner |
|---|---|---|
| Munich | 13 December 2021 | Vix, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GAN BEE HA ET AL: "The multifaceted nature of antimicrobial peptides: current synthetic chemistry approaches and future directions", CHEMICAL SOCIETY REVIEWS, vol. 50, no. 13, 27 May 2021 (2021-05-27), pages 7820-7880, XP055871390, UK ISSN: 0306-0012, DOI: 10.1039/D0CS00729C * see abstract and pages 7834-7837 * ----- | 1-15 | |
| Y | WO 2015/166272 A2 (IONTAS LTD [GB]) 5 November 2015 (2015-11-05) * see claims 1-8 * ----- | 1-15 | |
| Y | WO 2011/116138 A2 (UNIV WAYNE STATE [US]; CUNNINGHAM PHILIP R [US]; COLANGELO WES [US]) 22 September 2011 (2011-09-22) * see claims * ----- | 1-15 | |
| Y | SAADET ALBAYRAK GURALP ET AL: "From Design to Screening: A New Antimicrobial Peptide Discovery Pipeline", PLOS ONE, vol. 8, no. 3, 19 March 2013 (2013-03-19), pages 1-7, XP055326042, DOI: 10.1371/journal.pone.0059305 * the whole document * ----- | 1-15 | **TECHNICAL FIELDS SEARCHED (IPC)** |

-/--

The present search report has been drawn up for all claims

| Place of search | Date of completion of the search | Examiner |
|---|---|---|
| Munich | 13 December 2021 | Vix, Olivier |

EPO FORM 1503 03.82 (P04C01)

**page 3 of 4**

Europäisches
Patentamt

European
Patent Office

Office européen
des brevets

## EUROPEAN SEARCH REPORT

**Application Number**

**EP 21 18 2971**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PUENTES PAOLA RUIZ ET AL: "Design, Screening, and Testing of Non-Rational Peptide Libraries with Antimicrobial Activity: In Silico and Experimental Approaches", ANTIBIOTICS, vol. 9, no. 12, 30 November 2020 (2020-11-30), page 854, XP055872367, DOI: 10.3390/antibiotics9120854 * the whole document * | 1-15 | |
| A | WO 2020/148420 A1 (CENTRE NAT RECH SCIENT [FR]; UNIV STRASBOURG [FR] ET AL.) 23 July 2020 (2020-07-23) * The query sequence SEQ ID NO:10 has 100 % identity (100 % similarity) over 18 positions in a common overlap (range (q:s): 1-18:1-18) with subject GSP:BIB12186 (length: 19) from WO2020148420-A1 published on 2020-07-23. * | 1-15 | |
| A | US 2013/029879 A1 (SHETTY RESHMA [US] ET AL) 31 January 2013 (2013-01-31) * the whole document * | 1-15 | TECHNICAL FIELDS SEARCHED (IPC) |
| A,D | HAMDI AMEL ET AL: "Yeast two-hybrid methods and their applications in drug discovery", TRENDS IN PHARMACOLOGICAL SCIENCES., vol. 33, no. 2, 1 February 2012 (2012-02-01), pages 109-118, XP055802844, GB ISSN: 0165-6147, DOI: 10.1016/j.tips.2011.10.008 * the whole document * | 1-15 | |

The present search report has been drawn up for all claims

| Place of search | Date of completion of the search | Examiner |
|---|---|---|
| Munich | 13 December 2021 | Vix, Olivier |

## ANNEX TO THE EUROPEAN SEARCH REPORT
## ON EUROPEAN PATENT APPLICATION NO.

EP 21 18 2971

This annex lists the patent family members relating to the patent documents cited in the above-mentioned European search report.
The members are as contained in the European Patent Office EDP file on
The European Patent Office is in no way liable for these particulars which are merely given for the purpose of information.

13-12-2021

| Patent document cited in search report | | Publication date | Patent family member(s) | | Publication date |
|---|---|---|---|---|---|
| WO 2020123387 | A1 | 18-06-2020 | AU | 2019398113 A1 | 24-06-2021 |
| | | | CA | 3122692 A1 | 18-06-2020 |
| | | | EP | 3908115 A1 | 17-11-2021 |
| | | | WO | 2020123387 A1 | 18-06-2020 |
| WO 2015166272 | A2 | 05-11-2015 | AU | 2015255013 A1 | 17-11-2016 |
| | | | BR | 112016025352 A2 | 17-10-2017 |
| | | | CA | 2947513 A1 | 05-11-2015 |
| | | | CN | 106574264 A | 19-04-2017 |
| | | | DK | 3137602 T3 | 25-02-2019 |
| | | | DK | 3461893 T3 | 10-08-2020 |
| | | | EA | 201691990 A1 | 31-05-2017 |
| | | | EP | 3137602 A2 | 08-03-2017 |
| | | | EP | 3461893 A1 | 03-04-2019 |
| | | | EP | 3741852 A2 | 25-11-2020 |
| | | | ES | 2712632 T3 | 14-05-2019 |
| | | | HR | P20190187 T1 | 22-03-2019 |
| | | | JP | 6440824 B2 | 19-12-2018 |
| | | | JP | 6738397 B2 | 12-08-2020 |
| | | | JP | 2017518362 A | 06-07-2017 |
| | | | JP | 2019023240 A | 14-02-2019 |
| | | | JP | 2020171319 A | 22-10-2020 |
| | | | PT | 3137602 T | 27-02-2019 |
| | | | PT | 3461893 T | 14-09-2020 |
| | | | SG | 11201609123R A | 29-11-2016 |
| | | | US | 2017073664 A1 | 16-03-2017 |
| | | | US | 2020165595 A1 | 28-05-2020 |
| | | | US | 2020165596 A1 | 28-05-2020 |
| | | | US | 2020165597 A1 | 28-05-2020 |
| | | | WO | 2015166272 A2 | 05-11-2015 |
| WO 2011116138 | A2 | 22-09-2011 | US | 2013150260 A1 | 13-06-2013 |
| | | | WO | 2011116138 A2 | 22-09-2011 |
| WO 2020148420 | A1 | 23-07-2020 | EP | 3911664 A1 | 24-11-2021 |
| | | | WO | 2020148420 A1 | 23-07-2020 |
| US 2013029879 | A1 | 31-01-2013 | US | 2013029879 A1 | 31-01-2013 |
| | | | US | 2017074889 A1 | 16-03-2017 |

EPO FORM P0459

For more details about this annex : see Official Journal of the European Patent Office, No. 12/82

**EP 4 112 728 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NUIJENS et al.** Natural Occurring and Engineered Enzymes for Peptide Ligation and Cyclization. *Front. Chem.,* vol. 7, 829
- **ENNO KLUSSMANN ; JOHN SCOTT.** Protein-Protein Interactions as New Drug Targets. Springer, 2008
- **SCOTT et al.** Small molecules, big targets: drug discovery faces the protein-protein interaction challenge. *Nature Reviews Drug Discovery,* 2016, vol. 15, 533-550
- **REZWAN ; AUERBACH.** Yeast "N"-hybrid systems for protein-protein and drug-protein interaction discovery. *Methods (San Diego, Calif.),* 2012, vol. 57 (4), 423-429
- **HAMDI ; COLAS.** Yeast two-hybrid methods and their applications in drug discovery. *Trends Pharmacol Sci,* February 2012, vol. 33 (2), 109-18

- **LAURENT et al.** Efforts to make and apply humanized yeast. *Briefings in Functional Genomics,* March 2016, vol. 15 (2), 155-163
- **MARTIN-YKEN.** Yeast-Based Biosensors: Current Applications and New Developments. *Biosensors,* 2020, vol. 10 (5), 51
- *Breast Cancer Research,* 2011, vol. 13
- **SLANZI et al.** In vitro Models of Neurodegenerative Diseases. *Front. Cell Dev. Biol.,* 13 May 2020
- **BUTTERWORTH, P.J. ; D.L. NELSON ; M.C. COX.** Lehninger: principles of biochemistry. W.H. Freeman % Co, 2004, 1119, , 17
- *Cell Biochem. Funct.,* 2005, vol. 23, 293-294
- **KYTEDOOLITTLE KYTE, J. ; DOOLITTLE, R.F.** A simple method for displaying the hydrophatic character of a protein. *J Mol. Biol.,* 1982, vol. 157, 2015-132